# EUROPEAN PATENT SPECIFICATION

(11) **EP 3 243 533 B1**
(45) Date of publication and mention of the grant of the patent: **17.06.2020**
(21) Application number: 17177810.3
(22) Date of filing: 10.10.2012
(51) Int. Cl.: A61L 15/28, A61L 27/20, A61L 31/04, C08B 37/00, D02G 3/44, C08L 5/08

(54) **THREADS OF CROSS-LINKED HYALURONIC ACID AND METHODS OF USE THEREOF**
STRÄNGE AUS VERNETZTER HYALURONSÄURE UND VERFAHREN ZUR VERWENDUNG DAVON
FILS D'ACIDE HYALURONIQUE RÉTICULÉ ET LEURS PROCÉDÉS D'UTILISATION

(30) Priority: 11.10.2011 US 201161545962 P; 07.12.2011 US 201161568077 P; 09.05.2012 US 201261644945 P
(43) Date of publication of application: 15.11.2017
(62) Divisional of application: 12773485.3
(73) Proprietor: Allergan Holdings France S.A.S., 92400 Courbevoie (FR)
(72) Inventor: FERMANIAN, Sara, San Francisco, California 94109 (US); HORNE, Kenneth N., San Francisco, California 94123 (US); SHENOY, Vivek, Redwood City, California 94061 (US); RAJADAS, Jayakumar, Cupertino, California 94014 (US); PRIOR, Jeff, Redwood City, California 94065 (US); JAYAKUMAR, Naveen, Cupertino, California 95014 (US); GURTNER, Geoffrey C., Stanford, California 94305-5148 (US)
(74) Representative: Hoffmann Eitle

(56) References cited:
- WO-A1-2011/109129
- US-A- 4 716 154

## Description

### Field

This disclosure relates generally to threads of hyaluronic acid, methods of making such threads and uses thereof, for example, in aesthetic applications (e.g., facial contouring, dermal filling), surgery (e.g., sutures), drug delivery, negative pressure wound therapy, moist wound dressing, and the like.

### Background

Hyaluronic acid (HA) is a linear polysaccharide (i.e., non-sulfated glycosaminoglycan) consisting of a repeated disaccharide unit of alternately bonded β-D-N-acetylglucosamine and β-D-glucuronic acid which can be depicted by the formula: 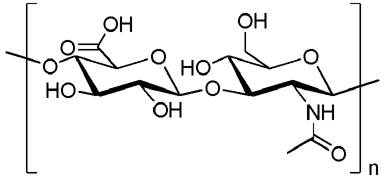 where n is the number of repeating units. Hyaluronic acid is sometimes referred to by the nomenclature (-4GlcUAβ1-3GlcNAcβ1-)ₙ) and is a chief component of the extracellular matrix found, for example, in connective, epithelial and neural tissue. Natural hyaluronic acid is highly biocompatible because of its lack of species and organ specificity and is often used as a biomaterial in tissue engineering and as a common ingredient in soft tissue augmentation products.

Natural hyaluronic acid has poor *in vivo* stability due to rapid enzymatic degradation and hydrolysis and, accordingly, various chemically modified forms of hyaluronic acid (e.g., cross-linked forms, ionically modified forms, esterified forms, etc.) have been synthesized to address this problem. Currently, hyaluronic acid or cross-linked versions thereof are used in various gel forms, for example as soft tissue augmentation products, adhesion barriers, and the like.

However, issues exist with the use of gels of hyaluronic acid or its cross-linked versions as soft tissue augmentation products. First, the force required to dispense gels of hyaluronic acid or its cross-linked versions is non-linear which can cause an initial ejection of a "glob" of gel that many physicians report when using injectable hyaluronic acid gels. Second, precisely dispensing hyaluronic gels to specific locations can be difficult because such gels have little mechanical strength. Further, the gel will occupy the space of least resistance which makes its use in many applications (e.g., treatment of fine wrinkles) problematic as the gel will often migrate into unintended spatial areas rendering the cosmetic procedure difficult and possibly even dangerous. Third, many common soft tissue augmentation products which are injected into the treatment site as a liquid or a gel, such as Restylane® (hyaluronic acid), Juvederm® (hyaluronic acid) Radiesse® (calcium hydroxyl apatite), Sculptra® (poly-L-lactic acid) and Perlane® (hyaluronic acid), are capable of migration and/or causing unsightly "lumps" which are painful to treat. Fourth, these soft tissue augmentation products are not recommended for use around the eyes as migration from the injection site can cause blindness, tissue necrosis, and in rare cases even stroke. Finally, clinicians also find performing lip augmentations using these fillers time consuming and patients find treatments in this area so painful that nerve blocks are routinely performed.

Accordingly, threaded forms of hyaluronic acid and its cross-linked versions have been developed that can be dispensed uniformly to specific locations regardless of tissue resistance, and without the risk of migration at implantation. These threaded forms are beneficial because they have improved tensile strength and greater ease of delivery.

Due to the significant therapeutic potential of threaded forms of hyaluronic acid, there remains a need to develop reaction conditions and manufacturing protocols that will yield improved threads and soft tissue augmentation products having superior physical properties.

US 4 716 154 A discloses vitreous humor substitute intended for ophthalmological uses and consisting of a gel of crosslinked hyaluronic acid.

WO 2011/109129 A1 discloses threads of hyaluronic acid, methods of making such threads and uses thereof.

### Summary

Threads have been developed comprising cross-linked hyaluronic acid. It has been surprisingly found that alterations, as described throughout, in the relative amounts of the components, reaction conditions, covalent modification of the hyaluronic acid, and manufacturing protocols can have significant effects upon certain properties of threads comprising cross-linked hyaluronic acid.

In one aspect, there is provided a composition comprising (i) at least 5 weight % hyaluronic acid, wherein the hyaluronic acid is substantially cross-linked with at least about 15 mole % of a butanediol diglycidyl ether (BDDE) derivative relative to the repeating disaccharide unit of the hyaluronic acid, and (ii) a binder which is noncross-linked hyaluronic acid, wherein the substantially cross-linked hyaluronic acid is cross-linked with at least 30 weight % to 50 weight % of BDDE relative to the weight of the hyaluronic acid. The threads described herein can be prepared using a composition comprising (i) at least 5 weight % hyaluronic acid, wherein the hyaluronic acid is substantially cross-linked with at least about 15 mole % of a butanediol diglycidyl ether (BDDE) derivative relative to the repeating disaccharide unit of the hyaluronic acid, and (ii) a binder which is noncross-linked hyaluronic acid, wherein the substantially cross-linked hyaluronic acid is cross-linked with at least 30 weight % to 50 weight % of BDDE relative to the weight of the hyaluronic acid. It has been discovered that the concentration of cross-linking agent, i.e. BDDE, used to prepare the substantially cross-linked hyaluronic acid, is used to tune and/or improve certain physical properties of the thread. The composition comprises at least 5% hyaluronic acid before cross-linking, such as 8%, 10% or 12% hyaluronic acid.

Further, threads as described herein comprise both cross-linked and noncross-linked hyaluronic acid. Surprisingly, the relative concentrations of these two components was discovered to impact certain physical properties of the threads, which ultimately led to an increased *in vivo* effectiveness as soft tissue augmentation products.

In addition, as is detailed herein, various aspects of the thread manufacturing process (e.g., rinsing, deaeration, extrusion, and drying of precursor gels, as well as the terminal sterilization of the dry threads) can be altered to produce threads having improved physical characteristics. Specifically, threads comprising cross-linked hyaluronic acid have been prepared with significant cross-linking (e.g., at least about 15% BDDE derivative) relative to the repeating disaccharide unit of the hyaluronic acid. This increased cross-linking within the cross-linked hyaluronic acid composition is contemplated to result in threads with a longer half-life *in vivo.*

The hyaluronic acid threads described herein possess an increased *in vivo* half-life when compared to the hyaluronic acid threads described previously in the art. When implanted in the dorsum of rabbits, sterilized threads described in the art were fully resorbed within 30 days whereas the threads described herein were still present at 3 months or longer in some cases.

In one of its method embodiments, there is provided a method of treating a wrinkle in a subject in need thereof. In such an aspect, the thread is inserted into the skin of a patient adjacent to or under the wrinkle. The thread is then applied under the wrinkle, thereby treating the wrinkle. In one embodiment, upon exposure to body fluids or by manually hydrating, the thread expands upon hydration and such expansion is typically sufficient to fill-in the wrinkle. It is advantageous to have a thread expand upon hydration because the invasiveness of the insertion profile is minimized, however, threads designed to not expand can also be used to treat the wrinkle.

Also encompassed is a kit of parts comprising the thread. The kit further comprises a means for delivering the thread. The means for delivery can either be a syringe or a needle.

### Brief Description of the Drawings

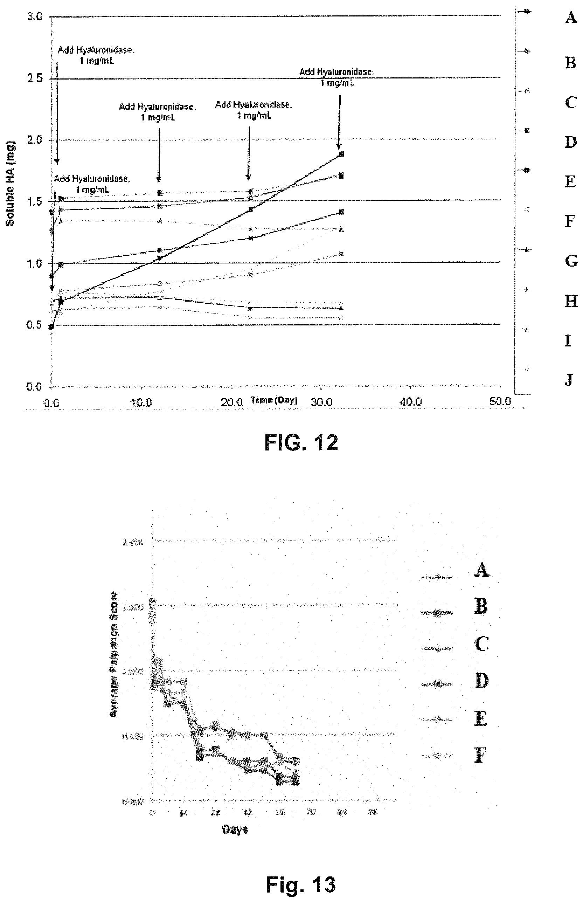

Certain aspects are best understood from the following detailed description when read in conjunction with the accompanying drawings. It is emphasized that, according to common practice, the various features of the drawings are not to-scale. On the contrary, the dimensions of the various features are arbitrarily expanded or reduced for clarity. Included in the drawings are the following figures:
**Fig. 1** shows a schematic of hyaluronic acid cross-linked with butanediol diglycidyl ether (BDDE).
**Fig. 2** illustrates a thread attached to the proximal end of a needle, in its entirety (N = needle; T = thread).
**Figs. 3A and 3B** show a needle attached to the thread (N = needle; T = thread). **Fig. 3A** illustrates a close-up view of a thread inserted into the inner-diameter of a needle; and **Fig. 3B** illustrates a close-up view of the proximal end of a solid needle with the thread overlapping the needle.

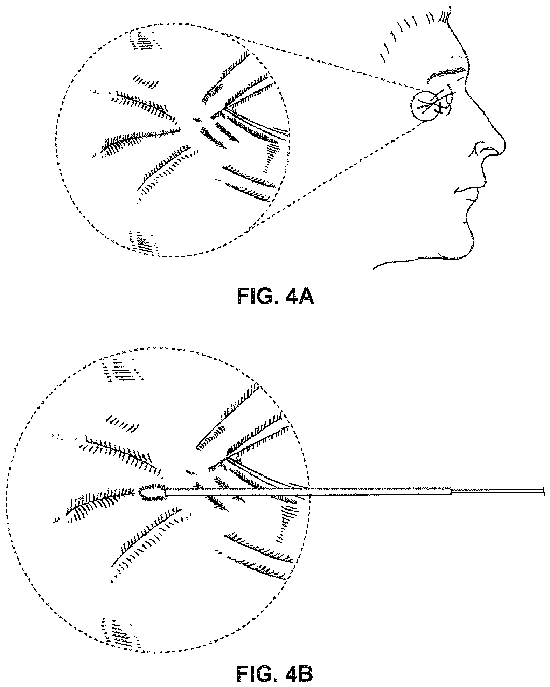

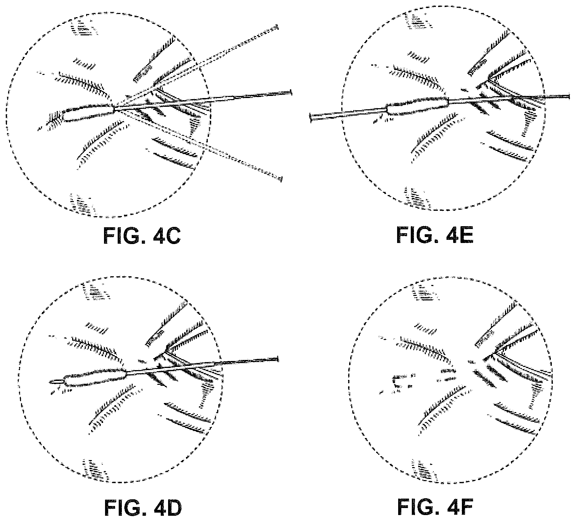

**Figs. 4A-4F** show treatment of a wrinkle. **Fig. 4A** illustrates a fine, facial wrinkle in the peri-orbital region of a human; **Fig. 4B** illustrates a needle and thread being inserted into the skin of the wrinkle at the medial margin; **Fig. 4C** illustrates the needle being adjusted to traverse beneath the wrinkle; **Fig. 4D** illustrates the needle exiting at the lateral margin of the wrinkle; **Fig. 4E** illustrates the needle having pulled the thread into the location it previously occupied beneath the wrinkle; and **Fig. 4F** illustrates the thread implanted beneath the wrinkle, with excess thread having been cut off.
**Figs. 5A-5C** show treatment of a wrinkle. **Fig. 5A** illustrates a cross-sectional view of a fold or a wrinkle; **Fig. 5B** illustrates a thread implanted beneath a wrinkle that is not yet hydrated; and **Fig. 5C** illustrates a thread implanted beneath a wrinkle that is fully hydrated and has flattened the surface appearance of the wrinkle.
**Fig. 6** illustrates how a needle and thread could be used to place a thread in a specific, linear location to promote nerve or vessel regrowth in a specific line.
**Fig. 7A** shows placement of threads in a relatively parallel orientation for facial contouring in the tear trough (Thread 1, 2, 3, 4, 5, and 6). This figure also shows placement of the thread for facial contouring of the nasolabial fold (Thread 7 and 8). **Fig. 7B** shows an alternative placement of the threads for facial contouring in the tear trough (Thread 1, 2, 3, 4, 5, 6, 7, and 8).

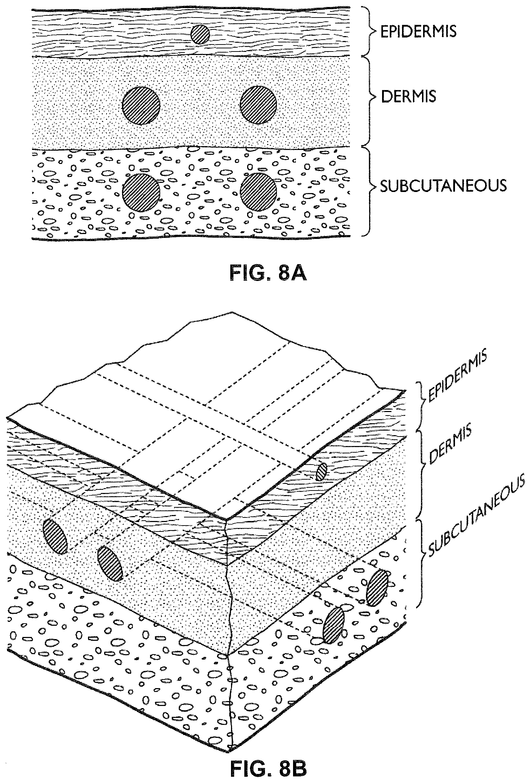

**Figs. 8A and 8B** show a schematic of the contemplated microanatomy of a thread implanted into a patient both in cross-section of the skin and three-dimensional cross-section.
**Figs. 9A, 9B, 9C, 9D and 9E** show (at 14x magnification) a histological cross section of a rabbit, one month (9A), two months (9B), three months (9C), six months (9D) and nine months (9E) after being treated with threads prepared from 8/40@15/20 thread composition (phosphate buffer wash; 15% HA solids, 20% of HA is noncross-linked binder in water). Details of the histological studies illustrated in **Figs. 9A, 9B, 9C, 9D and 9E** can be found in Example 9.
**Fig. 10** shows a gross dissection of a rabbit one month after being treated with threads prepared from 8/40@15/20 thread composition (phosphate buffer wash; 15% HA solids, 20% of HA is noncross-linked HA binder). Details of the dissection illustrated in **Fig. 10** can be found in Example 10.
**Fig 11** shows a gross dissection of a rabbit two months after being treated with threads prepared from 8/40@15/20 thread composition (phosphate buffer wash; 15% HA solids, 20% of HA is noncross-linked HA binder). Details of this dissection illustrated in **Fig. 11** can be found in Example 10.
   The following thread nomenclature is used in **Figs. 12 and 13** to describe the gel compositions: AA/BB@XX/YY, wherein (AA) is the weight % of hyaluronic acid relative to the weight of the cross-linking solution; (BB) is the weight % of BDDE relative to the weight of the hyaluronic acid; (XX) is the weight % of cross-linked and noncross-linked hyaluronic acid "solids" relative to the weight of the composition (pre-extrusion); and (YY) is the weight % of noncross-linked hyaluronic acid relative to the weight of total cross-linked and noncross-linked hyaluronic acid "solids".
**Fig. 12** shows the results of a thread degradation study with hyaluronidase (1 mg/mL). Details of these enzymatic degradations represented in **Fig. 12** can be found in Example 7. For threads A-F: A = 10/40@15/20 CaCl₂; B = 10/40@15/40; C = 10/40@15/20; D = 10/40@10/50; E = 8/40@15/20 H₂O wash; F = 8/40@15/20; G = Control for 10/40@15/20 CaCl₂; H = Control for 10/40@15/40; I = Control for 10/40@15/20; J = Control for 8/40@15/20. Vertical arrows correspond to the addition of fresh 1 mg/mL of hyaluronidase.
**Fig. 13** shows average palpation scores for exemplary threads as described hereins. Details of these palpation studies represented in **Fig. 13** can be found in Example 8. For threads A-F: A = 8/40@15/20; B = 8/40@15/20; C = 10/40@10/50; D = 10/40@15/20; E = 10/40@15/20; F = 10/40@15/40; Needle = pre-sized (20G); terminally sterilized (20kGy); thread size ∼0.01 inches.

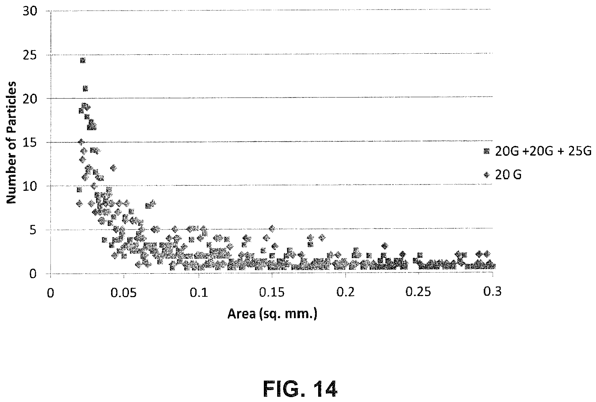

**Fig. 14** shows the particle size distribution of particles up to 0.3 square mm in diameter of the gel with one sizing and multiple sizing steps.

### Detailed Description

Described herein are threads of substantially cross-linked hyaluronic acid, the compositions from which they are made, methods for their preparation and uses thereof and to specific shapes formed there from. However, the following terms will first be defined.

It must be noted that as used herein and in the appended claims, the singular forms "a", "an", and "the" include plural referents unless the context clearly dictates otherwise. Thus, for example, reference to "a thread" includes a plurality of threads.

### 1. Definitions

Unless defined otherwise, all technical and scientific terms used herein have the same meaning as commonly understood by one of ordinary skill in the art to which this disclosure belongs. As used herein the following terms have the following meanings.

The term "hyaluronic acid" or "HA" refers to the polymer having the formula: 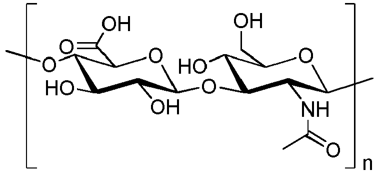 where n is the number of repeating units. All sources of hyaluronic acid are useful, including bacterial and avian sources. Hyaluronic acids useful have a molecular weight of from about 0.5 MDa (mega Dalton) to about 3.0 MDa. In some embodiments, the molecular weight is from about 0.6 MDa to about 2.6 MDa and in yet another embodiment, the molecular weight is from about 1.4 MDa to about 1.7 MDa. In some embodiments, the molecular weight is about 0.7 MDa and in yet another embodiment, the molecular weight is about 1.7 MDa. In some embodiments, the molecular weight is about 2.7 MDa.

At least a portion of the thread as described herein is cross-linked. The term "cross-linked" is intended to refer to two or more polymer chains of hyaluronic acid which have been covalently bonded via a cross-linking agent. Such cross-linking is differentiated from intermolecular or intramolecular dehydration which results in lactone, anhydride, or ester formation within a single polymer chain or between two or more chains. Although, it is contemplated that intramolecular cross-linking may also occur in the threads as described herein. The term "cross-linked" is also intended to refer to hyaluronic acid covalently linked to a BDDE derivative. In some embodiments, the term "cross-linked" also refers to covalently modified hyaluronic acid.

"Cross-linking agents" contain at least two reactive functional groups that create covalent bonds between two or more molecules. The cross-linking agents can be homobifunctional (i.e. have two reactive ends that are identical) or heterobifunctional (i.e. have two different reactive ends). The cross-linking agents to be used in the present disclosure should comprise complimentary functional groups to that of hyaluronic acid such that the cross-linking reaction can proceed. In one embodiment, the cross-linking does not form esterified hyaluronic acid. Suitable cross-linking agents include, by way of example only, butanediol diglycidyl ether (BDDE), divinyl sulfone (DVS), or 1-ethyl-3-(3-dimethylaminopropyl) carbodimide hydrochloride (EDC), or a combination thereof. In one embodiment, the cross-linking agent is BDDE. In one embodiment, the cross-linking agent is not a photocurable cross-linking agent.

As used herein, the term "BDDE derivative" refers to a form of BDDE wherein one or both epoxides of BDDE have reacted with hyaluronic acid. BDDE has the following chemical structure:

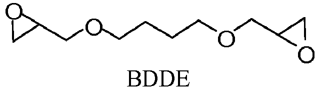

One example of a BDDE derivative of hyaluronic acid is shown below.

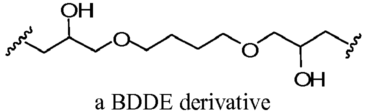

The BDDE derivative of hyaluronic acid, as shown above, can be covalently bound to hyaluronic acid at both ends with both epoxides having been reacted. Additional BDDE derivatives of hyaluronic acid are contemplated herein. For example, certain BDDE derivatives of hyaluronic acid can be covalently bound at both ends between two separate hyaluronic acid polymers (i.e., cross-linked), while other BDDE derivatives can be covalently bound at both ends within a single hyaluronic acid polymer. Also contemplated are BDDE derivatives that are covalently bound at one or both ends to a hydroxyl group from one or more additional BDDE derivatives that are themselves covalently bound to hyaluronic acid.

Also contemplated are BDDE derivatives that that are covalently bound to hyaluronic acid at just one end. For example, one of the epoxide rings can be opened by covalent attachment to a single stretch of a hyaluronic acid polymer while the other epoxide ring can remain closed (i.e., unreacted). It is further contemplated that, within the cross-linked hyaluronic acid compositions, the concentration of such BDDE derivatives with an unreacted epoxide is sufficiently low so as not to affect the biocompatibility of threads prepared from such compositions. Further contemplated is a BDDE derivative in which one of the epoxide rings has been opened by covalent attachment to a single stretch of hyaluronic acid polymer while the other epoxide ring has been opened by hydrolysis. However, it is contemplated that the cross-linked hyaluronic acid compositions comprise at least about 2 mole % BDDE (with respect to the disaccharide monomer) which is covalently bound at both ends between two separate hyaluronic acid polymers.

As used herein, the term "binder" refers to a naturally occurring or synthetic substance which provides uniform consistency and/or cohesion in the composition comprising the cross-linked hyaluronic acid, which when extruded, forms a thread. In one embodiment, the binder is noncross-linked hyaluronic acid. In another embodiment, the binder is selected from a group consisting of sugars and polysaccharides such as sucrose, maltose, chondroitin sulfate, dermatan sulfate, heparin, chitosan, cellulose, gelatin, collagen, acacia, starch, PVP (polyvinyl pyrrolidone), HPC (hydroxypropyl cellulose), HPMC (hydroxypropyl methylcellulose), PEG, PLGA (poly(lactic-co-glycolic acid), carboxy methyl cellulose, ethylcellulose, gelatin polyethylene oxide, dextrin, magnesium aluminum silicate, polymethacrylates, and the like.

As used herein, the term "skin" refers to the three layers: the epidermis, the dermis, and the hypodermis or the deeper subcutaneous tissue.

As used herein, the terms "smoother," "smooth," and "smoothness" refer to the property of a thread that provides decreased drag when pulled through tissue. The more smooth the thread, the less drag when pulled through the skin.

As used herein, the term "thread" refers to a long, thin, flexible form of a material. The thread as described herein can have a variety of shapes in the cross-section which are discussed below.

The term "ultimate tensile strength" is intended to refer to the tensile strength of the thread which has been normalized with respect to cross-sectional area. The term "tensile strength" is intended to refer to the maximum stress a thread can withstand without failing when subjected to tension. In one embodiment, it is contemplated that the ultimate tensile strength is sufficient to pull the thread through the skin and manipulate it once in the skin such that the integrity of the thread is not substantially compromised by, for example, breaking or segmenting.

It is contemplated that threads as described herein preferably have an ultimate tensile strength of about 3 kpsi ("kilopounds per square inch") or greater, or 5 kpsi or greater, or 10 kpsi or greater, or 15 kpsi or greater or 20 kpsi or greater or 50 kpsi or greater or 75 kpsi or greater. In some embodiments, the threads have a tensile strength of about 0.4 lbf (pound force) or greater, or 0.6 lbf or greater, or 0.8 lbf or greater, or 1.0 lbf or greater, or 1.1 lbf or greater. In some embodiments, the threads have a tensile strength of about 0.7 lbf.

In some embodiments, tensile strength may be measured by using a force gauge and measuring the peak force required to break the thread. Of approximately 9 thread lots tested, the average tensile strength was about 0.71 pounds force using a 20 gauge extrusion nozzle.

The term "percent moisture" is intended to refer to the total percent of water by weight. In one embodiment, the percent moisture of the thread is about 30% or less, or alternatively, about 15% or less, or alternatively, about 10% or less. This can typically be measured by Karl Fisher titration.

The threads as described herein can be made into a variety of shapes. The term "substantially cylindrical" refers to a thread wherein the cross-section of the thread is round. The term "substantially" as used to refer to shapes of the threads means that at least 50% of the thread has the approximate shape described. The term substantially is also used to encompass threads which have a variety shapes along the length of the thread. For example, a thread could be substantially cylindrical but the ends of the thread may be tapered. The substantially cylindrical threads can be provided when the contact angle of the gel composition and the substrate on which it is extruded have an equilibrium contact angle of greater than about 90 degrees.

The term "substantially D-shaped" refers to a thread wherein the cross-section is D-shaped or substantially semi-circular. The substantially D-shaped threads have one flat side and one substantially round side. The substantially D-shaped threads can be provided when the contact angle of the gel composition and the substrate on which it is extruded have an equilibrium contact angle of about 90 degrees.

The term "substantially ribbon-shaped" refers to a thread wherein the thickness of the thread is less than about 50% of the width of the thread. In some embodiments, the cross-section is substantially rectangular. The ribbon-shaped threads can be provided when the contact angle of the gel composition and the substrate on which it is extruded have an equilibrium contact angle of less than about 90 degrees. Alternatively, the ribbon-shaped threads can be formed by cutting a wet gel to achieve the desired cross-sectional shape. "Ribbon-shaped" may also include shapes that are substantially ellipsoidal. The term "substantially ellipsoidal" refers to a thread wherein the cross-section is substantially oblong or elliptical.

The term "therapeutic agent" can include one or more therapeutic agents. In still other of the above embodiments, the therapeutic agent is an anesthetic, including but not limited to, lidocaine, xylocaine, novocaine, benzocaine, prilocaine, ripivacaine, propofol, or combinations thereof. In still other of the above embodiments, the therapeutic agent includes, but is not limited to, epinephrine, ephedrine, aminophylline, theophylline or combinations thereof. In still other of the above embodiments, the therapeutic agent is botulism toxin. In still other of the above embodiments, the therapeutic agent is laminin-511. In still other of the above embodiments, the therapeutic agent is glucosamine, which can be used, for example, in the treatment of regenerative joint disease. In still other of the above embodiments, the therapeutic agent is an antioxidant, including but not limited to, vitamin E or all-trans retinoic acid such as retinol. In still other of the above embodiments, the therapeutic agent includes stem cells. In still other of the above embodiments, the therapeutic agent is insulin, a growth factor such as, for example, NGF (nerve growth factor),BDNF (brain-derived neurotrophic factor), PDGF (platelet-derived growth factor) or Purmorphamine Deferoxamine NGF (nerve growth factor), dexamethasone, ascorbic acid, 5-azacytidine, 4,6-disubstituted pyrrolopyrimidine, cardiogenols, cDNA, DNA, RNAi, BMP-4 (bone morphogenetic protein-4), BMP-2 (bone morphogenetic protein-2), an antibiotic agent such as, for example, β lactams, quinolones including fluoroquinolones, aminoglycosides or macrolides, an anti-fibrotic agent, including but not limited to, hepatocyte growth factor or Pirfenidone, an anti-scarring agent, such as, for example, anti-TGF-b2 monoclonal antibody (rhAnti-TGF-b2 mAb), a peptide such as, for example, GHK copper binding peptide, a tissue regeneration agent, a steroid, fibronectin, a cytokine, an analgesic such as, for example, Tapentadol HCl, opiates, (e.g., morphine, codone, oxycodone, etc.) an antiseptic, alpha- beta or gamma-interferon, EPO, glucagons, calcitonin, heparin, interleukin-1, interleukin-2, filgrastim, a protein, HGH, luteinizing hormone, atrial natriuretic factor, Factor VIII, Factor IX, or a follicle-stimulating hormone.

The term "diagnostic agent" refers to an agent which is used as part of a diagnostic test (e.g., a fluorescent dye to be used for viewing the thread *in vivo*)*.* In one embodiment, the diagnostic agent is soluble TB (tuberculosis) protein.

The term "lubricity-enhancing agent" is intended to refer to a substance or solution which when contacted with the dry thread, acts to lubricate the dry thread. A lubricity-enhancing agent can comprise, for example, water and/or an alcohol, an aqueous buffer, and may further comprise additional agents such as polyethylene glycol, hyaluronic acid, and/or collagen.

The term "biodegradation impeding agent" is intended to refer to a biocompatible substance that slows or prevents the *in vivo* degradation of the thread. For example, a biodegradation impeding agent can include hydrophobic agents (e.g., lipids) or sacrificial biodegradation agents (e.g., sugars).

The term "failure load" is intended to refer to the maximum force which, when applied to the thread, causes the thread to fail. By "failing," it meant that the thread can break or segment or otherwise lose structural integrity. In some embodiments, the failure load is about 0.1 pounds or 0.22 kilograms or greater.

The term "firm" is intended to refer to a cohesive material that maintains its form in an unconstrained environment (ie as opposed to a flowable/amorphous material) and demonstrates some degree of structural integrity under compression. A gelatin cube is an example of a firm gel.

The term "aqueous gel composition" or "gel composition" or "gel mixture" is intended to refer to an aqueous composition comprising water, hyaluronic acid, and a cross-linking agent and/or cross-linked hyaluronic acid. In some embodiments, the composition may further comprise a buffer such that that the pH of the solution changes very little with the addition of components of the composition. In these embodiments, the composition is referred to as an aqueous buffered gel composition. The pH of the buffered gel composition is typically from about 7 to about 13. In certain embodiments the pH is about 7. In certain embodiments, the pH is higher at about 9 or about 10. In some embodiments, the pH can be adjusted by adding an appropriate amount of a suitable base, such as Na₂CO₃ or NaOH. In some embodiments, the aqueous gel buffered composition comprises phosphate buffered saline. In some embodiments, the aqueous gel buffered composition comprises tris(hydroxymethyl)aminomethane (Tris), which has the formula (HOCH₂)₃CNH₂. In some embodiments, additional solutes are added to adjust the osmolarity and ion concentrations, such as sodium chloride, calcium chloride, and/or potassium chloride.

The term "buffer" is intended to refer to a solution that stabilizes pH, wherein the solution comprises a mixture of a weak acid and its conjugate base or a weak base and its conjugate acid. Buffer solutions include, but are not limited to, 2-amino-2-methyl-1,3-propanediol, 2-amino-2-methyl-1-propanol, L-(+)-tartaric acid, D-(-)-tartaric acid, ACES, ADA, acetic acid, ammonium acetate, ammonium bicarbonate, ammonium citrate, ammonium formate, ammonium oxalate, ammonium phosphate, ammonium sodium phosphate, ammonium sulfate, ammonium tartrate, BES, BICINE, BIS-TRIS, bicarbonate, boric acid, CAPS, CHES, calcium acetate, calcium carbonate, calcium citrate, citrate, citric acid, diethanolamine, EPP, ethylenediaminetetraacetic acid disodium salt, formic acid solution, Gly-Gly-Gly, Gly-Gly, glycine, HEPES, imidazole, lithium acetate, lithium citrate, MES, MOPS, magnesium acetate, magnesium citrate, magnesium formate, magnesium phosphate, oxalic acid, PIPES, phosphate buffered saline, piperazine potassium D-tartrate, potassium acetate, potassium bicarbonate, potassium carbonate, potassium chloride, potassium citrate, potassium formate, potassium oxalate, potassium phosphate, potassium phthalate, potassium sodium tartrate, potassium tetraborate, potassium tetraoxalate dehydrate, propionic acid solution, STE buffer solution, sodium 5,5-diethylbarbiturate, sodium acetate, sodium bicarbonate, sodium bitartrate monohydrate, sodium carbonate, sodium citrate, sodium chloride, sodium formate, sodium oxalate, sodium phosphate, sodium pyrophosphate, sodium tartrate, sodium tetraborate, TAPS, TES, TNT, TRIS-glycine, TRIS-acetate, TRIS buffered saline, TRIS-HCl, TRIS phosphate-EDTA, tricine, triethanolamine, triethylamine, triethylammonium acetate, triethylammonium phosphate, trimethylammonium acetate, trimethylammonium phosphate, Trizma® acetate, Trizma® base, Trizma® carbonate, Trizma® hydrochloride or Trizma® maleate.

The term "aqueous solvent" is intended to refer to a non-toxic, non-immunogenic aqueous composition. The aqueous solvent can be water and/or an alcohol, and may further comprise buffers, salts (e.g., CaCl₂) and other such non-reactive solutes.

The term "contact angle" or "equilibrium contact angle" refers to a measure of a liquid's affinity for a solid and quantifies the degree of a liquid drop's spread when placed on the solid. In the case as described herein, the liquid is the aqueous gel composition and the rigid or solid surface is the substrate on which the composition is extruded. The contact angle is a measure of the angle that the edge of an ideal drop makes with a flat surface. The lower that the contact angle is, the greater attraction between the surface and the liquid. For example, water spreads almost completely on glass and has a very low contact angle of nearly 0 degrees. Mercury, in contrast, beads up and spreads very little; its contact angle is very large.

### 2. Threads and Methods of Preparing Threads

As mentioned above, it has been surprisingly found that alterations of the relative amounts of the components, reaction conditions, covalent modification of the hyaluronic acid, and/or manufacturing protocols can have notable effects upon certain properties of threads comprising cross-linked hyaluronic acid. Accordingly, such threads with improved characteristics have been developed and methods of their preparation are described herein. Such threads are contemplated to be smoother. Some of the threads that are described below are stronger. They have greater tensile strengths and improved capacities to absorb water. It is thus contemplated that the threads described herein are easier for the clinician to handle and implant into to a patient.

### Preparation of Cross-Linked Hyaluronic Acid

Threads comprising cross-linked hyaluronic acid have been prepared according to methods described herein having increased ratios of cross-linking agent (e.g., BDDE derivatives) relative to the repeating disaccharide unit of the hyaluronic acid.

Generally, hyaluronic acid (HA) used herein has a molecular weight of from about 0.5 MDa (mega Dalton) to about 3.0 MDa. In some embodiments, the molecular weight is from about 0.6 MDa to about 2.6 MDa, and in yet another embodiment the molecular weight is from about 1.4 MDa to about 1.7 MDa. In some embodiments, the molecular weight is about 0.7 MDa, and in yet another embodiment the molecular weight is about 1.7 MDa. In some embodiments, the molecular weight is about 2.7 MDa.

In one aspect, there are provided compositions comprising cross-linked hyaluronic acid formed under aqueous conditions. In certain embodiments, such aqueous compositions form gels. In certain embodiments, the hyaluronic acid is hydrated for between about one minute and about 60 minutes prior to cross-linking. In other embodiments, the hyaluronic acid is hydrated for between about one hour and about 12 hours prior to cross-linking. In certain embodiments, the hyaluronic acid is hydrated for about one hour and in yet another embodiment the hyaluronic acid is allowed to hydrate for about two hours prior to cross-linking. In certain embodiments, the hyaluronic acid is hydrated for about three hours and in yet another embodiment the hyaluronic acid is allowed to hydrate for four hours prior to cross-linking.

Prior to addition of the HA, the aqueous solution is adjusted to the desired pH. In one embodiment, the aqueous solution has a pH > about 7. In certain embodiments, the solution has a pH of about 9, or about 10, or about 11, or about 12 or about 13, or greater than 13. Typically, the solution comprises water and can optionally comprise phosphate buffered saline (PBS) or tris(hydroxymethyl)aminomethane (Tris) buffer. The buffer can be selected based on the desired pH of the composition. For example, PBS can be used for compositions at a pH of about 7, whereas Tris can be used for compositions having a higher pH of about 9 or about 10. In some embodiments, the pH is from between about 9 and about 13. In some embodiments, the pH is at least about 13. In some embodiments, the pH is adjusted with the appropriate amount of a suitable base, such as Na₂CO₃ or NaOH to reach the desired pH. In some embodiments, the concentration of base is from about 0.00001 M to about 0.5 M. In some embodiments, the concentration of base is from about 0.1 M to about 0.25 M. In some embodiments, the concentration of base is about 0.2 M.

It has been surprisingly found that the concentration of hyaluronic acid used during the cross-linking contributes to the quality of the compositions comprising cross-linked hyaluronic acid, and ultimately improves certain properties of threads that are prepared from such compositions. For example, the gels become increasingly firm when the concentration of hyaluronic acid used during cross-linking is at least about 5%. Further, it has been found that the gel swelling ratio in water can be increased by decreasing the concentration of hyaluronic acid used during the cross-linking. In one embodiment, the composition during the cross-linking comprises from about 1 weight % to about 25 weight % hyaluronic acid, before cross-linking. In another embodiment, the composition during the cross-linking comprises about 14 weight % hyaluronic acid, before cross-linking. In another embodiment, the composition during the cross-linking comprises about 12 weight % hyaluronic acid, before cross-linking. In another embodiment, the composition during the cross-linking comprises about 8 weight % hyaluronic acid, before cross-linking. In another embodiment, the composition during the cross-linking comprises about 5 weight % hyaluronic acid, before cross-linking.

In an alternative embodiment, the cross-linking is performed neat, i.e., without a solvent. Therefore, in certain embodiments, neat BDDE is contacted with dry hyaluronic acid to provide the cross-linked hyaluronic acid. The composition can then be hydrated with the desired amount of aqueous medium to provide the gel composition.

Compositions comprising cross-linked hyaluronic acid are formed when hyaluronic acid is contacted with a cross-linking agent. The cross-linking agent to be used in the present disclosure is a butanediol diglycidyl ether (BDDE) derivative. There is provided a composition comprising substantially cross-linked hyaluronic acid, wherein hyaluronic acid is cross-linked or covalently modified with a BDDE derivative.

It has also been discovered that the concentration of cross-linking agent, e.g., BDDE, used during cross-linking contributes to the quality of the compositions comprising cross-linked hyaluronic acid and, ultimately, to improve certain properties of the threads that are prepared from such compositions. The amount of BDDE used is sufficient to produce a composition comprising at least 15 mole % of a BDDE derivative relative to the repeating disaccharide unit of the hyaluronic acid. In one embodiment, the composition comprises from about 15 mole % to about 25% mole percent, or about 17 mole % to about 20 mole % of the BDDE derivative, or about 18 mole % to about 19 mole %. In one embodiment, the composition comprises 18.75% of the BDDE derivative.

The amount of BDDE selected will be sufficient enough to provide a firm composition. For example, the gels become increasingly firm when the concentration of BDDE used during cross-linking is at least about 10 weight % relative to the weight of the hyaluronic acid. The amount of BDDE used during cross-linking, upon formation of the composition comprising cross-linked hyaluronic acid, may also be expressed as a weight % relative to the weight of the hyaluronic acid used during cross-linking. Between 30 weight % and 50 weight % BDDE is used relative to the weight of hyaluronic acid. In another embodiment, about 30% BDDE is used relative to the weight of hyaluronic acid. In another embodiment, about 40% BDDE is used relative to the weight of hyaluronic acid. In another embodiment, about 50% BDDE is used relative to the weight of hyaluronic acid.

In certain aspects, hyaluronic acid is cross-linked or covalently modified to form compositions comprising substantially cross-linked hyaluronic acid. In certain embodiments, the amount of cross-linking agent incorporated therein, or cross-link density, should be sufficiently high such that the thread formed thereby is elastomeric, however it should not be so high that the resulting thread becomes too rigid such that it cannot be moved within the skin during delivery when used as a soft tissue augmentation product. The appropriate stiffness or elastic modulus is determined by the intended use of the thread.

### Washing, Drying, and Formulating Cross-Linked Hyaluronic Acid

After the cross-linked hyaluronic acid has been prepared, any excess cross-linking agent can be washed away. Water rinsing alone is typically insufficient to remove all excess cross-linking agent. Water rinsing can also be followed with or replaced by rinsing with a buffer and/or alcohol solvent, such as ethanol to remove the unreacted BDDE. It is contemplated that multiple washings may be necessary to remove all or substantially all of the excess cross-linking agent. It is further contemplated that the gels may be cut into smaller pieces or extruded from a syringe to improve the efficiency of the washing steps.

In some embodiments, the hydrated or washed gel pieces have been sized. The sizing be accomplished by loading the gel into a syringe and extruding through a needle (typically a 20 gauge (G) blunt needle) or through a screen (e.g., 355 µm screen). More than one or even a series of sizing steps can be performed using the same or a different, typically a smaller, gauge needle or screen than the previous sizing step. For example, the gel can be first extruded through a 20G needle once or twice, and then optionally extruded through a 23G or a 25G needle one or more times. The more sizing steps implemented, the smoother the resultant thread. Such results would be beneficial as a smoother thread would ease delivery through the skin as the smoother thread would exhibit less drag. In addition, it is contemplated that additional sizing steps also may increase the tensile strength of the thread. For example, certain threads which have been sized once using a 20G needle have a tensile strength of from about 0.381 pounds to about 0.476 pounds, or of about 0.436 pounds. Certain threads which have been sized twice using a 20G needle have a tensile strength of from about 0.416 pounds to about 0.579 pounds, or of about 0.479 pounds. Certain threads which have been sized twice using a 20G needle and then once using a 25G needle have a tensile strength of from about 0.462 pounds to about 0.605 pounds, or of about 0.529 pounds.

Although the smoothness of the thread is enhanced by implementing more than one sizing step, the particle size of the gel is not substantially changed (Fig. 14). Thread swell ratio does not change substantially with increased sizing (between 1.66 and 1.70 for one, two and three sizing steps), although the dry diameter of the thread decreases slightly with increased sizing.

In one embodiment, the compositions comprising cross-linked hyaluronic acid, as described above, are substantially dried (e.g., dehydrated) before being further combined with binder. In one embodiment, the aqueous gel compositions comprising cross-linked hyaluronic acid, as described above, are substantially dried (e.g., dehydrated) before being further combined with noncross-linked hyaluronic acid. In some embodiments, drying is accomplished by airdrying or first decanting the solvent before air drying at ambient or elevated temperatures. In one embodiment, drying is accomplished by lyophilization. In one embodiment, drying is partial.

In one embodiment, the compositions comprising cross-linked hyaluronic acid, as described above, are isolated via precipitation from a suitable solvent, such as ethanol, before being further combined with binder. The precipitation can be implemented multiple, i.e., more than one, time. In some embodiments, the particle size of the gel isolated via precipitation are substantially the same size as, or smaller than, the particles of the lyophilized gel. In certain embodiments, the threads made from the gel isolated via precipitation have a small dried thread diameter (e.g., about 0.014"-0.015"), yet exhibit a faster rate of swelling, enhanced softness and larger swell diameter.

In one embodiment, the aqueous gel composition comprising cross-linked hyaluronic acid is dehydrated to remove about 25% of the water content by weight. In one embodiment, the aqueous gel composition comprising cross-linked hyaluronic acid is dehydrated to remove about 50% of the water content by weight. In one embodiment, the aqueous gel composition comprising cross-linked hyaluronic acid is dehydrated to remove about 75% of the water content by weight. In one embodiment, the aqueous gel composition comprising cross-linked hyaluronic acid is dehydrated to remove about 90% of the water content by weight.

It has been surprisingly found that the concentration of total HA solids, including cross-linked hyaluronic acid and noncross-linked binder, within the aqueous gel compositions prior to extrusion, improves certain properties of the threads as described herein such as smoothness and ease of handling either before or after extrusion.

The dried cross-linked hyaluronic acid composition, as described above, can be combined with water to form a substantially cross-linked hyaluronic acid gel, which is then formulated with a binder (i.e., noncross-linked hyaluronic acid) and optionally additive (e.g., a salt, excipient, lidocaine, or the like). The resulting formulated gel composition comprising cross-linked hyaluronic acid and noncross-linked hyaluronic acid as binder, can then be extruded into a wet thread which can then be dried.

In one embodiment, the cross-linked hyaluronic acid is present in the composition, before thread drying and with noncross-linked hyaluronic acid as binder, in an amount of from about 5 weight % to about 20 weight % based on the total weight of the composition. In still another embodiment, the cross-linked hyaluronic acid is present in the composition, before thread drying and with noncross-linked hyaluronic acid as binder, in an amount of from about 5 weight % to about 12 weight % or about 8 weight % to about 10 weight % based on the total weight of the composition, excluding moisture.

In one embodiment, there is provided a composition comprising the substantially cross-linked hyaluronic acid of any of the above embodiments, that has been dried and rehydrated, and further comprises a binder. The binder is noncross-linked hyaluronic acid. In one embodiment, the binder, i.e. noncross-linked hyaluronic acid, is provided as an aqueous solution. In one embodiment, the binder, i.e. noncross-linked hyaluronic acid, is provided as an aqueous solution further comprising a salt, such as CaCl₂. In one embodiment, the binder, i.e. noncross-linked hyaluronic acid, is provided as an aqueous solution further comprising 1 mM CaCl₂, 2.5 mM CaCl₂, 10 mM CaCl₂, or greater than 10 mM CaCl₂.

Yet another surprising finding has been that the quality of the dry threads as described herein is dependent, at least partially, upon the quantity of the total hyaluronic acid solids used to make the wet thread compositions, as described above. The "hyaluronic acid solids" include any combination of substantially cross-linked hyaluronic acid and/or noncross-linked hyaluronic acid (i.e., binder). In some embodiments, a minimum quantity of hyaluronic acid solids contributes to the quality of the dry threads, for example, by supporting the cross-sectional shape (e.g., relatively round diameter) of the wet threads used to make the dry threads. A minimum quantity of hyaluronic acid solids may further contribute to the quality of the dry threads, for example, by increasing the tensile strength of the dry threads and/or the swelling ratio by which the dry threads absorb water. A minimum quantity of hyaluronic acid solids may contribute still further to the quality of the dry threads by increasing the smoothness of the dry threads. Exemplary minimum quantities of hyaluronic acid solids are described below.

In one embodiment, the wet thread comprises from about 2% to about 50% hyaluronic acid solids. In one embodiment, the wet thread comprises from about 2% to about 40% hyaluronic acid solids. In one embodiment, the wet thread comprises from about 2% to about 20% hyaluronic acid solids. In one embodiment, the wet thread comprises at least 7% hyaluronic acid solids. In one embodiment, the wet thread comprises at least 10% hyaluronic acid solids. In one embodiment, the wet thread comprises at least 12% hyaluronic acid solids. In one embodiment, the wet thread comprises at least 15% hyaluronic acid solids. In one embodiment, the wet thread comprises at least 18% hyaluronic acid solids. In one embodiment, the wet thread comprises at least 20% hyaluronic acid solids. In one embodiment, the wet thread comprises at least 25% hyaluronic acid solids.

As described above, the hyaluronic acid solids that are used to make the wet and dry threads as described herein can include any combination of cross-linked hyaluronic acid and/or noncross-linked hyaluronic acid (i.e., binder). Adjustments in the quantity and/or ratio of cross-linked hyaluronic acid and/or noncross-linked hyaluronic acids can improve certain properties of the threads (e.g., the cross-sectional shape of the wet threads, the tensile strength of the dry threads, the swelling ratio by which the dry threads absorb water, the smoothness of the dry threads, the resistance of the dry threads to *in vitro* enzymatic digestion by hyaluronidase, and/or an increased *in vivo* half-life).

In one embodiment, the cross-linked hyaluronic acid is present in compositions used to make threads in an amount of at least 5 weight % based on the total weight of the composition. In another embodiment, the cross-linked hyaluronic acid is present in an about 14 weight %. In another embodiment, the cross-linked hyaluronic acid is present in an about 12 weight %. In another embodiment, the cross-linked hyaluronic acid is present in an about 8 weight %. In another embodiment, the cross-linked hyaluronic acid is present in an about 5 weight %.

In one embodiment, the noncross-linked hyaluronic acid is present in compositions used to make threads in an amount of from about 1 weight % to about 20 weight % based on the total weight of the composition. In another embodiment, the noncross-linked hyaluronic acid is present in an amount of from about 1 weight % to about 8 weight % based on the total weight of the composition. In another embodiment, the noncross-linked hyaluronic acid is present in an about 5 weight %. In another embodiment, the noncross-linked hyaluronic acid is present in an about 3 weight %. In another embodiment, the noncross-linked hyaluronic acid is present in an about 2 weight %.

In one embodiment, the compositions used to make threads comprise from about 5 weight % to about 15 weight % cross-linked hyaluronic acid and from about 2 weight % to about 8 weight % noncross-linked hyaluronic acid. In one embodiment, the composition comprises about 12 weight % cross-linked hyaluronic acid and about 3 weight % noncross-linked hyaluronic acid. In one embodiment, the composition comprises about 8 weight % cross-linked hyaluronic acid and about 2 weight % noncross-linked hyaluronic acid. In one embodiment, the composition comprises about 5 weight % cross-linked hyaluronic acid and about 5 weight % noncross-linked hyaluronic acid.

### Deaerating, Extruding, and Drying Gels Into Threads

In some embodiments, the aqueous gel composition comprising cross-linked and noncross-linked hyaluronic acid is deaerated (i.e., degassed), prior to extrusion to minimize air bubbles after extrusion. The degassing can also be done by using a syringe. It has been discovered that the tensile strength of threads generally improves upon deaeration of the compositions used to make the threads.

In some embodiments, the compositions used to make the threads are deaerated at least once. In some embodiments, the compositions used to make the threads are deaerated more than once. In some embodiments, the compositions used to make the threads are deaerated between two and ten times. In some embodiments, the compositions used to make the threads are deaerated twice. In some embodiments, the compositions used to make the threads are deaerated three times, four, five, six, seven, eight, nine, or ten times. In some embodiments, the compositions used to make the threads are deaerated at least ten times.

To form the thread, the aqueous gel composition comprising cross-linked and noncross-linked hyaluronic acid is typically extruded onto a substrate, as described below, to form a wet thread. The composition is extruded using a pressurized syringe affixed to a nozzle. The nozzle can have various geometries, such as various lengths, internal diameters and shapes.

Generally, it has been discovered that many of the properties of the dry thread compositions can be improved by increased nozzle size (e.g., width) during extrusion of the wet thread. Such improved properties in the threads can include, for example, the cross-sectional shape of the threads, the tensile strength of the threads, the swelling ratio by which the threads absorb water, the smoothness of the threads, the resistance of the dry threads to *in vitro* enzymatic digestion by hyaluronidase, and/or an increased *in vivo* half-life of the dry threads.

The nozzle may be circular or non-circular in shape, for example, a flattened shape or a "D" shape. The syringe nozzle may be anywhere from about a 15 gauge to a 25 gauge syringe nozzle. In some embodiments, the syringe nozzle is a 15 gauge nozzle, whereas in other embodiments the syringe nozzle is a 16 gauge nozzle. In some embodiments, the syringe nozzle is a 17 gauge nozzle, whereas in other embodiments the syringe nozzle is a 18 gauge nozzle. In some embodiments, the syringe nozzle is a 19 gauge nozzle, whereas in other embodiments the syringe nozzle is a 20 gauge nozzle. In some embodiments, the syringe nozzle is a 21 gauge nozzle, whereas in other embodiments the syringe nozzle is a 22 gauge nozzle. Typically, the pressure employed is from about 10 to about 2000 psi or from about 20 to about 240 psi. The pressure requirements are dictated by the nozzle geometry and other attributes such as consistency of the composition and desired flow rate. The pressure can be applied pneumatically, for example using ambient air or nitrogen, hydraulically, or mechanically. The speed at which the gel is extruded takes into consideration minimization of air bubbles in the length of the thread and maximization of a consistent uniform shape. Air bubbles can reduce the structural integrity of the thread by causing weak spots.

Pneumatic pressure and plate speed are not fixed but are instead adjusted and monitored in-process so that the gel is extruded in a continuous, linear manner. For a given lot of threads, the pneumatic pressure is first increased to the point of consistent but controllable gel flow. The plate speed is then continuously fine-tuned so that threads are extruded in a uniform linear manner. If the plate speed is too slow zig-zagged threads may result; too fast and threads may stretch leading to necking and/or breakage.

Various substrates are contemplated for use by methods as described herein. Substrates include by hydrophilic and hydrophobic substrates and may be selected from, but are not limited to, polytetrafluoroethylene (PTFE), expanded PTFE, nylon, polyethylene terephthalate (PET), polystyrene, silicon, polyurethane, and activated cellulose.

The substrate employed, along with the viscosity of the gel composition, dictates the general shape of the thread. For example, if the gel and the substrate have an equilibrium contact angle of less than 90 degrees, it is contemplated that the thread formed could be substantially ribbon-shaped. Further, if the gel and the substrate have an equilibrium contact angle of about 90 degrees, the thread formed could be substantially D-shaped. Still further, if the gel and the substrate have an equilibrium contact angle of greater than 90 degrees, then the thread formed could be substantially round. For example, a 10% 1.5 MDa gel will have a substantially circular cross-section (e.g., about 80% of a circle) when extruded on PTFE, while a 5% 1.5 MDa gel will form a flat ribbon when extruded on PTFE.

Alternative to pressurized extrusion, the gel composition can be rolled out into an elongated cylinder and/or cut into elongated strips before drying.

The wet thread is then dried to form a dry thread. Drying may be conducted under static conditions or, alternatively, with the assistance of a dynamic air flow (i.e., within a laminar flow hood). In some embodiments, yields of the threads improve with static drying. The drying step is required to form threads with a sufficient tensile strength, as discussed below. As the thread may lose some of its properties when exposed to heat in excess of water boiling temperature, it is preferred that the drying step be performed under ambient conditions. This drying procedure provides a thread with a higher tensile strength, such as, for example, an ultimate tensile strength of about 5 kpsi to 100 kpsi or 20 kpsi to 80 kpsi. In other words, the threads as described herein have a failure load of at least about 0.1 pounds or 0.22 kilograms.

The thread is allowed to dry for anywhere from about 30 minutes to about 72 hours to form threads having a diameter of from 0.05 mm to about 1.0 mm and having 10%-30% by weight hydration. In some embodiments, the thread can be dried for about 12 hours or about 24 hours. It is contemplated that the larger the molecular weight of HA employed or the more concentrated the HA in the composition, the longer the drying times that are required. Further, during the drying process, a non-thermal stimulus, such UV light, radiation, or a chemical initiator or a catalyst, may be employed to assist in the cross-linking reaction.

In some embodiments, after drying, the thread is washed with an aqueous or non-aqueous solvent, a gas or a supercritical fluid. In some instances, this washing removes excess cross-linking agent. The washing can be accomplished by a variety of methods, such as submersion in an aqueous solvent or by using a concurrent flow system by placing the thread in a trough at an incline and allowing an aqueous solvent to flow over the thread. Threads can also be suspended, for example vertically, and washed by dripping or flowing water down the length of the thread.

In one embodiment, water is used to wash the threads. In this embodiment, the water not only washes the threads to remove excess cross-linking agent, it also rehydrates the thread into a hydrated elastomeric state. In one embodiment, an antioxidant solution is used to wash the threads. For example, in one embodiment, a buffer solution comprising ascorbic acid, vitamin E and/or sodium phosphate is used to wash the threads. In one embodiment, a buffer solution comprising about 1 mM, or about 10 mM or about 100 mM, or about 1 M ascorbic acid is used to wash the threads.

The percent hydration of hyaluronic acid can range from about 1% to greater than about 1000% based on the total weight. The percent hydration of the thread of the present disclosure can be controlled by adjusting the percent hyaluronic acid in the gel and/or controlling the amount and type of cross-linking agent added. It is contemplated that a lower percent hydration thread would result in a thread with a higher tensile strength. In some embodiments, the thread has no more than about 30% percent, or no more than 15%, or no more than 10% by weight hydration based on the total weight. The percent hydration will be determined by the environment to which the thread is subjected to during or after the drying process.

It should be noted that the half-life of the hyaluronic acid thread *in vivo* can be controlled by controlling the thickness of the thread, the density, the degree of cross-linking, the molecular weight of the hyaluronic acid and the degree of hydration, which can then be further controlled by adjusting the amounts of hyaluronic acid and cross-linking agent both individually and relatively. It is contemplated that the threads disclosed herein can have an enhanced half-life *in vivo* of from about 1 month to up to about 12 months as compared to less than 1 day for natural hyaluronic acid. In certain embodiments, it is contemplated that the threads described herein have an *in vivo* half-life of at least 1 month. In certain embodiments, it is contemplated that the threads described herein have an *in vivo* half-life of at least 2 months. In certain embodiments, it is contemplated that the threads described herein have an *in vivo* half-life of at least 3 months. In certain embodiments, it is contemplated that the threads described herein have an *in vivo* half-life of at least 4 months. In certain embodiments, it is contemplated that the threads described herein have an *in vivo* half-life of at least 6 months. In certain embodiments, it is contemplated that the threads described herein have an *in vivo* half-life of at least 8 months. In certain embodiments, it is contemplated that the threads described herein have an *in vivo* half-life of at least 10 months. In certain embodiments, it is contemplated that the threads described herein have an *in vivo* half-life of at least 12 months. In certain embodiments, it is contemplated that the threads described herein have an *in vivo* half-life of at least 14 months. In certain embodiments, it is contemplated that the threads described herein have an *in vivo* half-life of at least 16 months. In certain embodiments, it is contemplated that the threads described herein have an *in vivo* half-life of at least 18 months.

It is contemplated that the threads described herein can be sterilized using typical sterilization methods known in the art, such as autoclave, ethyleneoxide, electron beam (e-beam), supercritical CO₂ (with peroxide), etc. For example, the threads as described herein can be sterilized using electron beam (e-beam) sterilization methods. In some embodiments, the threads are first washed in a buffer solution at high pH (i.e., pH 9 or pH 10). In some embodiments, the wash solutions further comprise ethanol, ascorbic acid, vitamin E and/or sodium phosphate.

Optionally and as necessary, the thread is mechanically stretched while hydrated, either soon after being hydrated or gradually before the first drying or after the rehydrating. The stretching or absence of stretching can provide a thread of the desired length and/or rehydration swelling volume. In some embodiments, the length of the thread can be from about 0.5 cm to about 15 cm. In another embodiment, the length of the thread can be from about 2 cm to about 12 cm. In another embodiment, the length of the thread can be from about 5 cm to about 10 cm.

After the thread is rehydrated it is allowed to dry again under ambient conditions for from anywhere from 30 minutes to about 72 hours. Upon drying, the thread, in some embodiments, cures to provide a more uniform surface of the thread.

This washing hydration/dehydration step can be performed multiple times to allow excess unreacted reagent to be washed from the thread or to continue to improve the degree of cross-linking or covalent modification. This is an improvement over methods such as the use of organic solvents to remove excess BDDE.

### 3. Thread Nomenclature

Threads as described herein are prepared from compositions comprising substantially cross-linked hyaluronic acid, and further comprising a binder, i.e. noncross-linked hyaluronic acid. Threads can be described according to the following nomenclature AA/BB@XX/YY, wherein AA/BB describes the initially formed composition comprising substantially cross-linked hyaluronic acid and XX/YY describes the composition with a binder, i.e. noncross-linked hyaluronic acid.

For example, a thread referred to as "10/40@15/20" refers to a composition of substantially cross-linked hyaluronic acid, wherein the cross-linking reaction is performed with 10 weight % hyaluronic acid relative to the weight of the solution (e.g., an aqueous solution), using 40 weight % cross-linker (e.g., BDDE) relative to the weight of the hyaluronic acid.

As referred to herein, AA of AA/BB@XX/YY is at least 2%. In some embodiments, AA is at least 5%. In some embodiments, AA is about 8%. In some embodiments, AA is about 10%. In some embodiments, AA is at least 10%. In some embodiments, AA is about 12%. In some embodiments, AA is at least 15%.

In some embodiments, BB of AA/BB@XX/YY is at least 10%. In some embodiments, BB is at least 20%. In some embodiments, BB is at least 30%. In some embodiments, BB is about 30%. In some embodiments, is at least 40%. In some embodiments, BB is about 40%. In some embodiments, BB is at least 50%. In some embodiments, BB is about 50%.

In some embodiments, AA/BB of AA/BB@XX/YY is about 8/10. In some embodiments, AA/BB is at least or about or exactly 8/20. In some embodiments, AA/BB is about 8/30. In some embodiments, AA/BB is about 8/40. In some embodiments, AA/BB is about 8/50. In some embodiments, AA/BB is about 10/10. In some embodiments, AA/BB is about 10/20. In some embodiments, AA/BB is about 10/30. In some embodiments, AA/BB is about 10/40. In some embodiments, AA/BB is about 10/50.

In some embodiments, one or more binding agents, including noncross-linked hyaluronic acid, are added to the compositions comprising cross-linked hyaluronic acid, and the resulting compositions are converted by additional methods described herein to provide novel threads. In some embodiments, the compositions comprising cross-linked hyaluronic acid further comprise noncross-linked hyaluronic acid. The added noncross-linked hyaluronic acid is optionally referred to herein as a "binder." The combination of cross-linked hyaluronic acid and noncross-linked hyaluronic acid, within the composition, is optionally referred to herein as "hyaluronic acid solids." As referred to herein, the XX of AA/BB@XX/YY refers to the weight % of total hyaluronic acid solids relative to the weight of the composition, wherein the hyaluronic acid solids includes both the substantially cross-linked hyaluronic acid and any noncross-linked hyaluronic acid. As referred to herein, the YY of AA/BB@XX/YY refers to the weight % of noncross-linked hyaluronic acid relative to the weight of total hyaluronic acid solids.

In some embodiments, XX of AA/BB@XX/YY is at least 2%. In some embodiments, XX is at least 5%. In some embodiments, XX is at least 10%. In some embodiments, XX is about 10%. In some embodiments, XX is at least 15%. In some embodiments, XX is about 15%. In some embodiments, XX is at least 20%. In some embodiments, XX is about 20%. In some embodiments, XX is at least 25%. In some embodiments, XX is about 25%.

In some embodiments, YY of AA/BB@XX/YY is at least 5%. In some embodiments, YY is at least 10%. In some embodiments, YY is at least 20%. In some embodiments, YY is about 20%. In some embodiments, YY is at least 30%. In some embodiments, YY is about 30%. In some embodiments, YY is at least 40%. In some embodiments, YY is about 40%. In some embodiments, YY is at least 50%. In some embodiments, YY is about 50%.

In some embodiments, AA/BB@XX/YY is 8/10@2/5, 8/20@5/10, 8/30@10/20, 8/40@15/20, 8/50@20/30, 10/10@20/40, 10/20@25/50, 10/30@20/40, 10/40@10/50, 10/50@20/40, and the like.

It has been shown that the threads provided using the AA/BB@XX/YY compositions disclosed herein exhibit an enhanced *in vivo* persistence, as well as other beneficial qualities.

In certain embodiments, disclosed herein are threads comprising substantially cross-linked hyaluronic acid, wherein the hyaluronic acid is substantially cross-linked with at least about 15 mole % of a butanediol diglycidyl ether (BDDE) derivative relative to the repeating disaccharide unit of the hyaluronic acid, and at least about 5% noncross-linked hyaluronic acid relative to the weight of total hyaluronic acid solids, wherein the cross-linked hyaluronic acid is present in an amount of from about 60 weight % to about 90 weight % based on the total weight of the thread excluding moisture and the noncross-linked hyaluronic acid is present in an amount of from about 10 weight % to about 40 weight % based on the total weight of the thread excluding moisture.

### 4. Modification of the Threads

In addition to washing the thread, it can also be further functionalized by adsorbing a sufficient amount of a member selected from the group consisting of a therapeutic agent, a diagnostic agent, a fibrogenesis-enhancing agent, a biodegradation impeding agent, a lubricity-enhancing agent and combinations thereof, optionally followed by re-drying the thread. Such therapeutic agents include antibacterials, anesthetics, dyes for viewing placement *in vivo,* and the like. In some embodiments, a dry or hydrated thread is coated to alter the properties with a bioabsorbable biopolymer, such as collagen, PEG, PLGA or a phase transfer Pluronic™ which can be introduced as a liquid and which solidifies *in vivo.*

In one embodiment, the thread can be coated to modulate the rate at which the thread is rehydrated. For example, the thread can be coated with a hydrophobic layer, such as a lipid. The thickness of the lipid layer can then be adjusted to achieve the desired rate of rehydration. In another embodiment, the thread can be coated with an aqueous composition of noncross-linked hyaluronic acid. This can be performed just prior to implantation of the thread to act as a lubricant. It is also contemplated that this coating with noncross-linked hyaluronic acid may slow the rate of hydration of the thread. In some embodiments, the thread is coated, either totally or in part, with the gel composition to form a layered material. Woven constructs, whether single layer or 3D, can be coated in their entirety to create weaves or meshes with altered physical properties from that of a free-woven mesh.

The threads as described herein can be braided, coiled, layered or woven. In some embodiments, braids may be formed from the threads described above. A braid can be formed by intertwining three or more threads wherein each thread is functionally equivalent in zigzagging forward through the overlapping mass of the others. The braids can be a flat, three-strand structure, or more complex braids can be constructed from an arbitrary (but usually odd) number of threads to create a wider range of structures, such as wider ribbon-like bands, hollow or solid cylindrical cords, or broad mats which resemble a rudimentary perpendicular weave.

In one embodiment, a plasticizer is added to adjust the stiffness of the thread. Alternatively, or in addition to, threads of varying stiffness may be weaved together to produce a braided thread or material having the desired stiffness.

In some embodiments, a three-dimensional structure may be constructed by weaving or wrapping or coiling or layering the threads described above. In other embodiments, a three-dimensional structure may be constructed by weaving or wrapping or coiling or layering the braids described above. In still other embodiments, a three-dimensional structure may be constructed by weaving or wrapping or coiling or layering the cords described above. In still other embodiments, a three-dimensional structure may be constructed by weaving or wrapping or coiling or layering the meshes described above.

In some embodiments, a three-dimensional, cylindrical implant is made of any of the threads is provided. An exemplary use for such an implant is for nipple reconstruction. In some embodiments, the threads used to make the cylindrical implant are cross-linked and include chrondrocyte adhesion compounds. In other embodiments, the cylindrical shape is provided by multiple, concentric coils of threads.

### Thread Embodiments

In one aspect, there is provided a dry thread comprising hyaluronic acid, wherein at least a portion of the hyaluronic acid is substantially cross-linked with at least about 15 mole % of a BDDE derivative relative to the repeating disaccharide unit of the hyaluronic acid. In another embodiment, the substantially cross-linked hyaluronic acid is cross-linked with from about 15 mole % to about 20 mole % of the BDDE derivative. In one embodiment, the substantially cross-linked hyaluronic acid is cross-linked with from about 16 mole % to about 19 mole % of the BDDE derivative.

In one embodiment, the cross-linked hyaluronic acid is present in an amount of from about 50 weight % to about 90 weight % based on the total weight of the dry thread. In another embodiment, the cross-linked hyaluronic acid is present in an amount of from about 60 weight % to about 80 weight % based on the total weight of the dry thread.

The dry thread further comprises a binder, i.e., noncross-linked hyaluronic acid. The noncross-linked hyaluronic acid may be present in an amount of from about 1 weight % to about 50 weight % based on the total weight of the dry thread. In one embodiment, the noncross-linked hyaluronic acid is present in an amount of from about 15 weight % to about 20 weight % based on the total weight of the dry thread.

In another embodiment, the dry thread has an ultimate tensile strength of from about 2 kpsi to about 20 kpsi. In one embodiment, the thread has an ultimate tensile strength of from about 4 kpsi to about 10 kpsi.

In another embodiment, the dry thread has a diameter of at least about 0.004 inches. In one embodiment, the dry thread has a diameter of from about 0.008 to about 0.018 inches.

In another embodiment, the dry thread has a weight/length ratio from about 1.5 to about 3.5 mg/inch.

In one embodiment, the dry thread has a failure load of about 0.3 pounds or greater. In another embodiment, the dry thread has a failure load of from about 0.3 to about 1.3 pounds.

In one embodiment, the dry thread further comprises a needle.

In yet another aspect, there is provided a dry thread comprising substantially cross-linked hyaluronic acid prepared by the steps of: a) forming a substantially cross-linked hyaluronic acid composition by contacting hyaluronic acid with BDDE; b) adding noncross-linked hyaluronic acid to the substantially cross-linked hyaluronic acid composition; c) extruding the substantially cross-linked hyaluronic acid composition to form a wet thread; and d) drying the wet thread to form a dry thread.

In one embodiment, from about 5 to about 15 weight % hyaluronic acid is contacted with from about 2 to about 8 weight % BDDE relative to the weight of the hyaluronic acid. In another embodiment, the hyaluronic acid is contacted with about 40 weight % BDDE relative to the weight of the hyaluronic acid. In one embodiment, the cross-linked hyaluronic acid is cross-linked with at least about 15 mole % of a BDDE derivative relative to the repeating disaccharide unit of the hyaluronic acid. In another embodiment, the cross-linked hyaluronic acid is cross-linked with from about 15 to about 25 mole % of the BDDE derivative relative to the repeating disaccharide unit of the hyaluronic acid. In one embodiment, the cross-linked hyaluronic acid is cross-linked with from about 17 to about 20 mole % of the BDDE derivative relative to the repeating disaccharide unit of the hyaluronic acid. In another embodiment, the cross-linked hyaluronic acid is cross-linked with at least about 12 weight % of the BDDE derivative relative to the weight of the hyaluronic acid.

In one embodiment, at least 5% hyaluronic acid is contacted with from about 2 to about 8 weight % BDDE relative to the weight of the hyaluronic acid composition. In another embodiment, about 8% hyaluronic acid is contacted with from about 2 to about 8 weight % BDDE relative to the weight of the hyaluronic acid composition. In one embodiment, about 10% hyaluronic acid is contacted with from about 2 to about 8 weight % BDDE relative to the weight of the hyaluronic acid composition.

In one embodiment, the composition formed by contacting hyaluronic acid with BDDE comprises cross-linked hyaluronic acid in an amount of from about 5 weight % to about 20 weight % hyaluronic acid based on the total weight of the composition. In another embodiment, the dry thread further comprises drying the composition formed by contacting hyaluronic acid with BDDE.

In one embodiment, from about 1 weight % to about 50 weight % noncross-linked hyaluronic acid is added, based on the total weight of the composition. In another embodiment, from about 2 weight % to about 15 weight % noncross-linked hyaluronic acid is added, based on the total weight of the composition. In another embodiment, about 3 weight % noncross-linked hyaluronic acid is added, based on the total weight of the composition.

In one embodiment, the dry thread has an ultimate tensile strength of from about 2 kpsi to about 20 kpsi. In one embodiment, the dry thread has an ultimate tensile strength of about 20 kpsi or greater.

In one embodiment, the dry thread has a failure load of about 0.3 pounds or greater. In one embodiment, the thread has a failure load of from about 0.3 to about 1.3 pounds.

In one embodiment, there is provided a thread according to any of the above embodiments, wherein the thread is terminally sterilized.

### 5. Methods of Using the Cross-Linked Hyaluronic Acid Threads

The threads, braids, cords, woven meshes or three-dimensional structures described herein can be used, for example, to fill wrinkles, to fill aneurysms, occlude blood flow to tumors, (i.e., tumor occlusion), in eye-lid surgery, in penile augmentation (e.g., for enlargement or for sensitivity reduction, i.e., pre-mature ejaculation treatment), inter-nasal (blood-brain barrier) delivery devices for diagnostic and/or therapeutic agents, corneal implants for drug delivery, nose augmentation or reconstruction, lip augmentation or reconstruction, facial augmentation or reconstruction, ear lobe augmentation or reconstruction, spinal implants (e.g., to support a bulging disc), root canal filler (medicated with therapeutic agent), glottal insufficiency, laser photorefractive therapy (e.g., hyaluronic acid thread/weave used as a cushion), scaffolding for organ regrowth, spinal cord treatment (BDNF and NGF), in Parkinson's disease (stereotactic delivery), precise delivery of therapeutic or diagnostic molecules, in pulp implantation, replacement pulp root canal treatment, shaped root canal system, negative pressure wound therapy, adhesion barriers (e.g., abdominal, pelvic, cardiac, spinal, and tendon adhesions), wound dressings, acellular dermal matrix, non-hydraulic drug delivery (e.g., pain (orthopedic), ophthalmic, etc.), luminal drug delivery (e.g., enlarged prostate, crohn's diease, vascular stenosis, etc.), and sustained, local drug delivery.

### Methods of Treating a Wrinkle

Tissue repair could prolong the "filler" effects of the thread when used to treat or fill a wrinkle *in vivo* far beyond the half-life of the hyaluronic acid-based thread as described herein. This is described in Example 11.

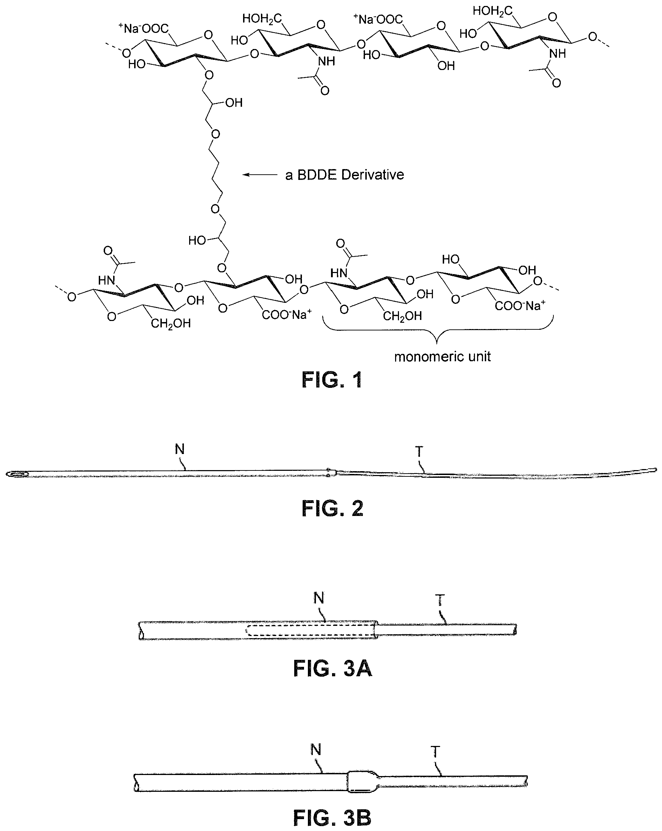

In some embodiments, there is provided a method of treating a wrinkle in a patient in need thereof by 1) inserting the thread as described herein into the dermis or subcutaneous space of the patient adjacent to or under the wrinkle; and 2) applying the thread adjacent to or under the wrinkle thereby treating the wrinkle. These steps can be performed at least once and up to 6 times to treat each wrinkle. In some embodiments, the thread is attached to the proximal end of a needle as shown in **Figs. 2, 3A** and **3B****.** The thread is inserted by a needle which needle is then removed. Optionally and as necessary, the thread is hydrated with water or saline, or by the fluids normally perfusing the surrounding tissue. Further, the remainder of the wrinkle can be filled with a biocompatible material such as a phase transfer Pluronic™ which can be introduced as a liquid and which solidifies *in vivo.* Alternatively, conventional hyaluronic acid gel can be introduced to fill the wrinkle. In either case, the formed web acts to maintain the biocompatible filler at the site of the wrinkle.

In some embodiments, a method of treating a wrinkle in a subject is provided. In some embodiments, the attending clinician may numb the treatment area according to procedures known in the art using a variety of anesthetics, including, but not limited to, topical lidocaine, ice or a block with lidocaine injection. For example, the wrinkle may be in the peri-orbital region as illustrated in **Fig. 4A****.** The thread may be attached to a needle as illustrated, for example, in **Figs. 2, 3A** and **3B****.** The distal end of the needle may be inserted through the skin surface of the subject into the dermis adjacent to or within the wrinkle as illustrated, for example, in **Fig. 4B****.** In some embodiments, the thread is inserted into the subcutaneous space instead of the dermis. The needle then may traverse the dermis or subcutaneous space of the subject beneath the wrinkle as illustrated, for example, in **Fig. 4C****.** The needle then may exit the skin of the subject at the opposite margin of the wrinkle, as illustrated, for example, in **Fig. 4D****.** The needle may then be pulled distally until it is removed from the subject such that the thread is pulled into the location previously occupied by the needle beneath the wrinkle, as illustrated, for example, in **Fig. 4E****.** Finally, excess thread is cut from the needle at the skin surface of the subject which leaves the thread implanted as illustrated, for example, in **Fig. 4F****.**

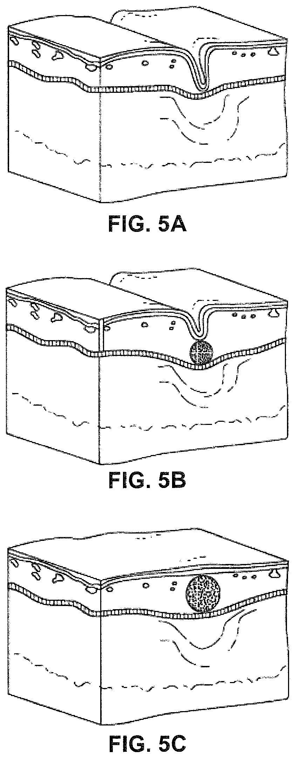

The method above may successfully treat wrinkles as shown in **Figs. 5A, 5B** and **5C****.** A typical wrinkle is illustrated in **Fig. 5A. Fig. 5B** illustrates a thread implanted beneath a wrinkle that is not yet hydrated. As the thread implanted beneath the wrinkle becomes fully hydrated the surface appearance of the wrinkle is concurrently flattened as illustrated in **Fig. 5C****.**

In some embodiments, the thread is manipulated in such a fashion such that one end of the thread is sufficiently hard such that the thread is used to penetrate the skin. This may be accomplished by coating the thread with a hardening material, such as a sugar coating, In another embodiment, the thread is coated in its entirety, for example with a sugar coating, to provide the thread with increased columnar strength.

### Facial Contouring

The threads as described herein are useful in facial contouring. What is meant by facial contouring is that the threads can be applied to any area of the face, neck, or chest that the patient desires to have augmented, including, by way of example only, the lips, the nasolabial fold, and tear trough.

Lip augmentation is a commonly desired aesthetic procedure. Typically, the aesthetic goal is fuller, plumper lips. Available treatment options for lip augmentation include temporary fillers such as Restylane® and Juvederm®, permanent fillers such as ArteFill®, Radiesse® and Goretex® implants, as well as surgical procedures. Areas of enhancement can include the vermillion border (or white roll) for lip effacement and contouring and the wet-dry mucosal junction for increasing fullness. Other techniques include more diffuse infiltration of the orbicularis oris muscle.

Lip contouring and augmentation by temporary soft tissue augmentation products is a popular, low risk option due to the minimal invasiveness and temporary nature of the procedure. The major shortcomings of soft tissue augmentation products currently used in lip procedures are that it is (a) painful, (b) difficult to consistently and homogenously inject the gel into the desired location, and (c) the gel can migrate over the lifetime of the implant causing the aesthetic results to change.

The present disclosure addresses the shortcomings described above. Beyond addressing the above-listed shortcomings for existing temporary soft tissue augmentation products described above, it has been found that the HA thread-based method of enhancing lip appearance is very quick. A typical patient may have 3 threads in their lip(s) in only 3 minutes. Current soft tissue augmentation product lip procedures can take 15 to 20 minutes.

In embodiments directed to facial contouring, the attending clinician may numb the treatment area according to procedures known in the art using a variety of anesthetics, including, but not limited to, topical lidocaine, ice, or a block with lidocaine injection. Threads made of HA (hyaluronic acid) can be attached to the proximal end of a needle and pulled into the lip. The needle can serve as a precise guide, and also be used to predict and correct the implant location prior to pulling the thread into the desired location. This precise delivery mechanism can be used to deliver threads along the vermillion border for contouring, superficially if desired, as well as at the wet-dry junction for plumping, deeper into the lip if desired.

It is contemplated that when the thread is used for facial contouring, any number of threads may be used depending on the desired effect and the size of the thread. For example, description of the procedure done for the lip augmentation and contouring is discussed below in Example 11.

It is has been surprisingly and unexpectedly found that that threads may be implanted in various tissue planes of the patient to provide a more natural look when performing facial contouring. For example, the threads may be implanted in a manner that forms a hammock in the desired location. Given the unique properties of the threads as described herein, the attending clinician may deposit or implant the threads in the epidermis, the dermis, and/or the subcutaneous layer.

This technique is enabled by the precision with which the threads can be placed, and their size relative to the dermis and underlying structures. Threads can impart different effects on facial features such as wrinkles, contours, folds and troughs depending on where they are implanted.

For example, recent clinical experience indicates that placing a thread (in this case, one that was approximately 008" in diameter) deeply, for example in the subcutaneous space, along the axis of a forehead wrinkle can help soften then appearance of the wrinkle that forms when the patient animates, by flexing their forehead - which would typically exacerbate the appearance of the wrinkle. These types of dynamic wrinkles are currently only well treated with Botox®, which has the undesirable effect of preventing the patient from expressing all facial expressions. Further, recent clinical experience shows that static wrinkles, ones that are visible in repose, can be effectively treated by placement of a thread (from .004 to .008" in diameter) superficially, for example within the dermis.

The technique of stratifying the thread implant tissue planes is also successfully used in improving the appearance of nasolabial folds (up to 4x .008" threads), glabellar lines, marionette lines, and lips.

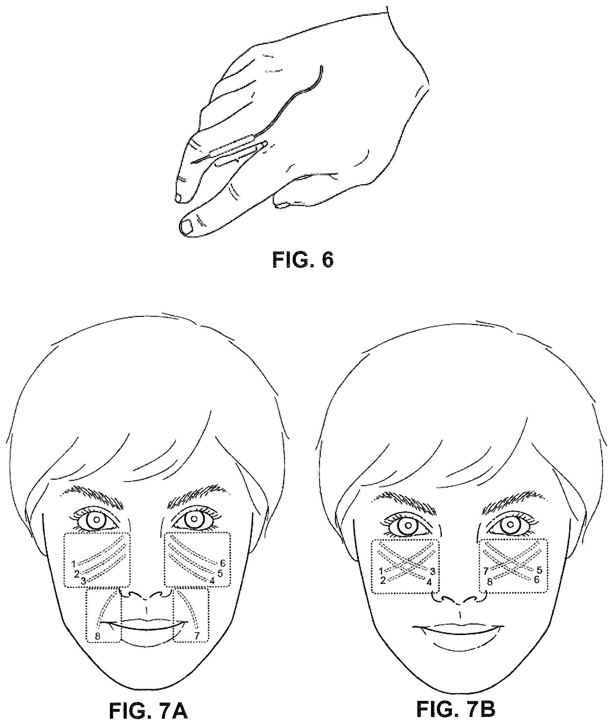

This is another technique that is enabled by the HA threads and their implantation method. To smooth the appearance of hollows or troughs such as the tear trough, or otherwise contour the face in areas such as the cheek bones, chin, for example, threads can be implanted in hatch (see, **Fig. 7A**) and/or cross-hatched patterns (see, **Fig. 7B**) to effect areas greater than the width of a single thread. As seen in **Figs. 7A** and **7B****,** two patients have their tear troughs effectively smoothed out by placing threads parallel in one case (**Fig. 7A**) and cross-hatched in another case (**Fig. 7B**). The cross-hatching could be done obliquely to the initial direction, as was the case in **Fig. 7B****,** or perpendicularly. Further, the hatches can be in different tissue planes as well.

In another embodiment of this technique, the hatching can be done obliquely to the directionality of the area being treated. For example, in **Fig. 7A** the threads are placed aligned to the axis of the tear trough. Instead, the threads could be placed obliquely to the axis of the tear trough to support the tissue in the area differently.

It is contemplated that implanting the threads in various planes may also be done in the treatment of wrinkles as described above.

### Wound Therapy

In some embodiments, the threads, braids, cords, woven meshes or three-dimensional structures described herein are used in wound dressings including negative pressure wound dressings.

In some embodiments, wound dressing remains in contact with the wound for at least 72 hours. In other embodiments, the negative pressure wound dressing remains in contact with the wound for at least 1 week. In still other embodiments, the wound dressing remains in contact with the wound for at least 2 weeks. In still other embodiments, the wound dressing remains in contact with the wound for at least 3 weeks. In still other embodiments, the wound dressing remains in contact with the wound for at least 4 weeks. In the above embodiments, it should be understood that granulation tissue is not retaining the threads, braids, cords, woven meshes or three-dimensional structures described herein as these components are fully absorbable. In some of these embodiments, the wound dressing is between about 1 cm and about 5 cm thick. Accordingly, in some of these embodiments, wound bed closure may be achieved without changing the dressing.

In some embodiments, the woven meshes described herein are used in wound dressings including negative pressure wound dressings. In other embodiments, the dressing include between 2 and about 10 layers of woven meshes.

In still other embodiments, the woven meshes comprise identical threads. In still other embodiments, the woven meshes comprise different threads.

In some embodiments, the woven meshes are between about 1 mm and about 2 mm thick when dry. In other embodiments, the woven meshes are between about 2 mm and about 4 mm thick when dry.

In some embodiments, the pore size of the woven mesh is between about 1 mm and about 10 mm in width. In other embodiments, the pore size of the woven mesh is between about 0.3 mm and about 0.6 mm in width. In still other embodiments, the pores of the woven mesh are aligned. In still other embodiments, the pores of the woven mesh are staggered. In still other embodiments, the woven meshes are collimated to create pores of desired size.

In some embodiments, the woven mesh is mechanically stable at a minimum vacuum level of about 75 mm Hg. In other embodiments, the woven mesh is mechanically stable at a vacuum up to about 150 mm Hg.

In some embodiments, the woven mesh includes collagen. In other embodiments, the dressing is attached to a polyurethane foam. In still other embodiments, the polyurethane foam is open celled. In still other embodiments, the dressing is attached to a thin film. In still other embodiments, the thin film is silicone or polyurethane. In still other embodiments, the dressing is attached to the thin film with a water soluble adhesive.

In some embodiments, the thread used in the dressing includes a therapeutic agent or a diagnostic agent.

In some embodiments, a negative pressure wound dressing (Johnson et al., U.S. Patent No. 7,070,584, Kemp et al., U.S. Patent No. 5,256,418, Chatelier et al., U.S. Patent No. 5,449383, Bennet et al., U.S. Patent No. 5,578,662, Yasukawa et al., U.S. Patent Nos. 5,629,186 5,780,281 and 7,611,500) is provided for use in vacuum induced healing of wounds, particularly open surface wounds (Zamierski U.S. Patent Nos. 4,969,880, 5,100,396, 5,261,893, 5,527,293 and 6,071,267 and Argenta et al., U.S. Patent Nos. 5,636,643 and 5,645,081). The dressing includes a pad which conforms to the wound location, an air-tight seal which is removably adhered to the pad, a negative pressure source in fluid communication with the pad and the threads, braids, cords, woven meshes or three-dimensional structures described herein attached to the wound contacting surface of the pad. The pad, seal, and vacuum source are implemented as described in the prior art.

In other embodiments, the threads, braids, cords, woven meshes or three-dimensional structures described herein are mechanically stable at a minimum vacuum level of about 75 mm Hg. In still other embodiments, the threads, braids, cords, woven meshes or three-dimensional structures described herein are mechanically stable at a vacuum up to about 150 mm Hg. In still other embodiments, the dressing includes at least one layer of woven mesh. In still other embodiments, the dressing include between 2 and about 10 layers of woven mesh.

In some embodiments a tube connects the pad to the negative pressure source. In still other embodiments, a removable canister is inserted between the pad and the negative pressure source and is in fluid communication with both the pad and the negative pressure source.

In some embodiments, the threads, braids, cords, woven meshes or three-dimensional structures described herein are not hydrated. Accordingly, in these embodiments, the dressing could absorb wound exudates when placed in contact with the wound. In other embodiments, the threads, braids, cords, woven meshes or three-dimensional structures described herein are hydrated. Accordingly, in these embodiments, the dressing could keep the wound moist when placed in contact with the wound.

In some embodiments, an input port attached to a fluid is connected with the pad. Accordingly, in these embodiments, fluid could be dispensed in the wound. In some embodiments, the fluid is saline. In other embodiments, the fluid contains diagnostic or therapeutic agents.

In some embodiments, the threads, braids, cords, woven meshes or three-dimensional structures described herein are used as adhesion barriers. In some embodiments, the woven meshes described herein are used in adhesion barriers. In some embodiments, the adhesion barriers can be implemented in conjunction with other types of adhesion barriers, such as liquids, gels, sprays, other films or solids, pharmaceuticals and/or cellular therapies.

### Hair Loss Treatment

In some embodiments, a method of treating hair loss in a subject is provided. A subject such as, for example, a male with typical male-pattern baldness may be treated with a thread, optionally attached to a needle, as illustrated, for example, in Figs. 2, 3A, and 3B. The distal end of the needle may be inserted into one of the hair lines. The needle then may traverse the area beneath the hairline of the subject and then may exit the skin of the subject. The needle may then be pulled distally until it is removed from the subject such that the thread is pulled into the location previously occupied by the needle. Finally, excess thread is cut from the needle at the skin surface of the subject which leaves the thread implanted. In some embodiments, the thread further comprises one or more compounds which promote hair growth.

### Additional Medical and Surgical Treatments

In some embodiments, the threads, braids, cords, woven meshes or three-dimensional structures described herein are used as soft tissue augmentation products in various aesthetic applications as described above. In other embodiments, the threads, braids, cords, woven meshes or three-dimensional structures described herein are used as sutures in various medical and/or surgical applications. In still other embodiments, the threads, braids, cords, woven meshes or three-dimensional structures described herein are used in ophthalmologic surgery, drug delivery, and intra-articular injection. In other embodiments, the threads, braids, cords, woven meshes or three-dimensional structures described herein are used as adhesion barriers, for example to treat abdominal, pelvic, cardiac, spinal, and/or tendon adhesions. In other embodiments, the threads, braids, cords, woven meshes or three-dimensional structures described herein are incorporated into an acellular dermal matrix. It is contemplated that an acellular dermal matrix integrated with the threads, braids, cords, woven meshes or three-dimensional structures described herein provide improved revascularization and/or biological integration. The threads, braids, cords, woven meshes or three-dimensional structures described herein can further comprise other regenerative biomaterials, biologics, and/or pharmacologics. In other embodiments, the threads, braids, cords, woven meshes or three-dimensional structures described herein are used as sustained, local drug delivery devices. In some embodiments, the drugs include reserpine, guanethidine, phenoxybenzamine and phentolamine, hexamethonium, 6-hydroxydopamine, tetrodotoxin, glutamate, etc. In other embodiments, the threads, braids, cords, woven meshes or three-dimensional structures described herein are used as passive drug eluting stents.

In some embodiments, a method for treating tumors in a subject in need thereof is provided. The thread may be attached to a needle as illustrated, for example, in **Figs. 2, 3A** and **3B****.** The distal end of the needle may be inserted into the tumor of the subject. The needle then may traverse the tumor and then may exit the tumor. The needle may then be pulled distally until it is removed from the tumor of the subject such that the thread is pulled into the location previously occupied by the needle. Finally, excess thread is cut from the needle which leaves the thread implanted in the tumor of the subject. In some of the above embodiments, the thread includes an anti-cancer agent. In some embodiments, the thread is cross-linked and includes Bcl-2 inhibitors.

In an exemplary embodiment, methods of the current disclosure may be used to treat pancreatic tumors. The pancreas may be accessed by surgery or minimally invasively methods such as by laparoscopy. The distal end of the needle may be inserted into the pancreatic tumor. The needle then may traverse the pancreatic tumor and then may exit the tumor. The needle may then be pulled distally until it is removed from the pancreatic tumor such that the thread is pulled into the location previously occupied by the needle. Finally, excess thread is cut from the needle which leaves the thread implanted in the pancreatic tumor. The process may be repeated any number of times to provide a pancreatic tumor which has been implanted with a number of threads. In some embodiments, the thread includes an anti-cancer agent.

In some embodiments, a method for treating a varicose vein in subject in need thereof is provided. The thread may be attached to a needle as illustrated, for example, in **Figs. 2, 3A** and **3B****.** The distal end of the needle may be inserted into the varicose vein of the subject. The needle then may traverse the varicose vein and then may exit the vein. The needle may then be pulled distally until it is removed from the varicose vein of the subject such that the thread is pulled into the location previously occupied by the needle. Finally, excess thread is cut from the needle which leaves the thread implanted in the varicose vein of the subject. In some embodiments, the needle is a flexible. In other embodiments, the thread coils when hydrated, more readily occluding the vessel.

In some embodiments, a method for nipple reconstruction is provided where a three-dimensional, cylindrical implant comprised of cross-linked threads is implanted underneath the skin. The implant may include therapeutic agents, for example chrondrocyte adhesion compounds.

In some embodiments, methods for nerve or vessel regrowth are provided. As illustrated in **Fig. 6****,** a needle can be used to place a thread in a specific line which could promote nerve or vessel regeneration.

### 6. Kits

Also proved herein is a kit of parts comprising a thread as described herein. In some embodiments, the kit comprises a thread and a means for delivering or implanting the thread to a patient. In one embodiment, the means for delivery to a patient is a syringe or a needle. In another embodiment, the means for delivery to a patient is an air gun. The size (or diameter) of the needle may depend on the use of the thread, and therefore also be based on the cross-sectional area of the thread used. The outer diameter of the needle or syringe may be greater than or equal to the cross-sectional area of the thread used to lessen the tensile requirement of the thread as it is being applied to the dermis. It is further contemplated that the outer diameter of the thread may be larger than the outer diameter of the needle. Skin is quite pliable so by having a smaller diameter needle can allow the puncture size to be small even with the use of a larger diameter thread. Further, the thickness of the thread would be different in the case where the thread is a suture in comparison to the treatment of fine lines and wrinkles where it may be that a thinner thread is used. More than one thread may also be attached to a single needle.

Further, the size of the delivery device, a needle, will be dependent on its intended use and the size of the thread. It is contemplated that for use in facial contouring and or wrinkle filling an about 0.006"(inch) to about 0.008" diameter thread or even an about 0.003" to about 0.004" diameter thread will be sufficient. However, in some embodiments, the thread is from about 0.003" to about 0.050", or from about 0.005" to about 0.030", or from about 0.005" to about 0.025". In some embodiments, the size of the thread is from about 0.010" to about 0.020" in diameter, or from about 0.011" to about 0.016".

The thread attachment to the needle can be either a mechanical attachment and/or with the use of an adhesive, such as cyanoacrylate. In one embodiment, the needle is stainless steel. In one embodiment, the thread woven or looped through holes in the proximal end of the needle, or alternatively, the thread wrapped around the proximal end of the needle, or alternatively, the thread threaded thru an eyelet of the needle and either tied or bonded with an adhesive to form a loop, or alternatively, the thread secured (either mechanically or bonded with an adhesive) within a hole in the proximal end of the needle. In another embodiment, the thread can be made to form a physical attachment to the needle during the drying process as the thread forms from the gel. For example, if a needle is used which has pores in the proximal end, the pores can fill with the gel during the extrusion process and the thread would be thus be secured upon drying. The needle can be rigid or flexible to enable the user to track the needle under the wrinkle within the skin. Further, the needle may be equipped with a ramp to guide the needle at a desired depth within the skin, and after needle insertion, the guide may be unclasped as the needle is brought through the skin surface. In some embodiments, the thread is attached to a needle.

It is further contemplated that the kit comprises a needle and the thread attached thereto, is packaged sterile, and intended for single use. Alternatively, a kit can comprise several needles, each with an attached thread. In an additional embodiment, a kit includes threads of different sizes to enable treatment options for the physician while minimizing the number of required needle sticks. In yet another embodiment, the kit includes threads and needles of different length and curved shapes to simplify implantation in areas that are difficult to access or treat with a straight needle, for example near the nose, around the eyes and the middle portion of the upper lip.

### Examples

The present disclosure is further defined by reference to the following examples. It will be apparent to those skilled in the art that many modifications, both to threads and methods, may be practiced without departing from the scope of the current disclosure. The hyaluronic acid and cross-linking agents are available from commercial sources.

### Example 1: Synthesis of a Cross-Linked Thread

Cross-linked hyaluronic acid threads can be made according to the following procedures.

### Cross-linking: preparation of cross-linked hyaluronic acid gel

Hyaluronic acid (HA) powder is hydrated in about 75% of a desired total volume of NaOH for about 30 minutes at about 50 °C in an appropriate container. The hydrated HA is then added to a syringe and mixed thoroughly (e.g., syringe-to-syringe about 20 times). Heating is continued for approximately 30 minutes.

Cross-linker (e.g., BDDE) is then dissolved in the remaining portion of the desired total volume of NaOH (i.e., about 25% of the desired total volume), added to the hydrated hyaluronic acid solution (dropwise or in one portion), mixed thoroughly (e.g., syringe-to-syringe about 20 times), heated for about 30 minutes, re-mixed (e.g., syringe-to-syringe about 20 times), and transferred to an appropriate container. Heating is then continued at about 50 °C for an additional 3 to 5 hours.

Various cross-linked gels were prepared with differing concentrations of components using the procedure described hereinabove.
- HA molecular weight (MDa): e.g., 0.7, 1.7, 2.7.
- HA hydration time: e.g., 0 minutes, 30 minutes, 1 hour, 2 hours, overnight.
- Reaction pH: 9-13.4 using, e.g., 0.00001-0.25 M NaOH.
- Cross-linking reaction time: e.g., 3-4.5 hours.
- HA concentration (% w/w HA:aqueous NaOH): e.g., 5, 6, 7, 8, 9, 10, 11, 12.
- BDDE concentration (% w/w BDDE:HA): e.g., 0.5, 2, 2.5, 5, 7.5, 8, 10, 20, 30, 40, 100.

### Rinsing/Optional Sizing

The cross-linked hyaluronic acid gel is rinsed to remove the sodium hydroxide and any unreacted BDDE. Water can also be removed, and the gels may be fragmented to facilitate the extrusion step and/or for rinsing efficiency. In such an instance, sizing is accomplished by cutting the gel into approximately 0.5 cm cubes. Rinsing is then achieved by rinsing with about 40 volumes of 10 mM sodium phosphate, at pH 6.0, three times for about 30 minutes each, rinsing with 100% ethanol six times for about 30 minutes, and then rinsing with water four times for about 30 minutes.

The swollen gel pieces can then be further sized into approximately 0.25 cm cubes, loaded into a syringe, and extruded through a 20 gauge (G) blunt needle. More than one sizing step can be performed using the same or a different, typically smaller, gauge needle (e.g., 20G, then two 25G sizing steps).

### Drying

The cross-linked hyaluronic acid gel is then diluted approximately 2:1 with water, loaded into a pan dried. The drying is accomplished by air drying under ambient conditions or lyophilization. Alternatively, the gel is isolated via precipitation from ethanol. The gel is either partially dried to a desired final concentration or dried completely.

### Formulating

The completely dried cross-linked hyaluronic acid gel is formulated as an aqueous composition to the desired final HA concentration (e.g., 2.5%, 5%, 7.5%, 10%, 12.5%, 15%, 20%). The partially dried cross-linked hyaluronic acid gel can be used as an aqueous composition in the formulation without further treatment.

The aqueous composition of cross-linked hyaluronic acid gel can then be further formulated with a binder such as noncross-linked hyaluronic acid. In such a case, noncross-linked hyaluronic acid is hydrated (e.g., overnight at 4 °C) at the desired final HA concentration (e.g., 2.5%, 5%, 7.5%, 10%, 12.5%, 15%, 20%). The binder is mixed with the cross-linked hyaluronic acid gel. Typical binders include noncross-linked hyaluronic acid, salts (e.g., CaCl₂), excipients, Lidocane, and the like.

The aqueous composition can comprise any aqueous medium, such as an acid, a base, a buffer or a salt. Buffers such as phosphate buffered saline can be used (e.g., 10 mM PBS at pH 7.4). Calcium chloride solutions can also be used (e.g., 1 mM, 2.5 mM, or 5 mM). Sodium hydroxide (NaOH) solutions may be used, (e.g., 0.1M, 0.2M, 0.3M, or 0.5M).

Compositions can be made with higher or lower concentrations of hyaluronic acid and cross-linked HA; the following three compositions are given as examples only.

In one composition, for example, the final extruded composition contained 12% (w/w) cross-linked hyaluronic acid and 3% (w/w) noncross-linked HA, wherein the cross-linked hyaluronic acid was derived from a cross-linking reaction with either 10% hyaluronic acid and 4% BDDE, or 8% hyaluronic acid and 3.2% BDDE.

In another composition, the final extruded composition contained 8% (w/w) cross-linked hyaluronic acid and 2% (w/w) noncross-linked HA, the cross-linked hyaluronic acid being derived from a cross-linking reaction with either 10% hyaluronic acid and 4% BDDE, or 8% hyaluronic acid and 3.2% BDDE.

In yet another composition, the final extruded composition contained 5% (w/w) cross-linked hyaluronic acid and 5% (w/w) noncross-linked HA, the cross-linked hyaluronic acid being derived from a cross-linking reaction with 10% hyaluronic acid and 4% BDDE.

### Extruding

The final gel formulations are then extruded onto a suitable surface to yield wet threads. Various nozzle sizes are used depending on the final desired thread thickness (e.g., 20G, 19G, 18G, 17G, 16G).

The final gel formulations are transferred to a pressurized extruder (e.g., EFD Model XL1500 pneumatic dispense machine). The nozzle of the extruder can have a tip ranging from a 15 gauge to about 25 gauge. The syringe pressure may be between about 10 psi and about 2000 psi, depending on the viscosity of the final gel formulation. For very viscous gel formulations, a pressure multiplier can be used.

The wet thread was then formed by extruding the final gel formulation onto a substrate by an extruder to achieve the desired wet thread thickness. For example, to achieve a similar dried diameter, one can use a 20 gauge needle for 15% hyaluronic acid compositions, or a 19 gauge needle for 10% hyaluronic acid compositions.

### Thread Drying

The wet thread can then be dried under ambient conditions to a percent hydration of less than about 30%, or less than about 15%, or less than about 10%, thus providing a dry thread. Optionally, the thread can be allowed to dry under a relative humidity of from about 20% to about 80% at a temperature of from about 20 °C to about 37 °C. For example, threads can be air-dried for two days at ambient conditions.

Optionally, prior to thread drying, the wet thread can be stretched to a desired length and reduced diameter prior to dying. The stretching can be by either hanging the thread by one end and applying weight to the opposing end, or by horizontally stretching the wet thread on a surface (either the same or different from the extrusion surface) and adhering the ends to the surface.

### Attaching to a Needle

The dry threads can be attached to a needle using known techniques (see, e.g., **Figs. 2, 3A** and **3B**).

### Sterilizing

The threads as described herein can be sterilized using electron beam (e-beam) sterilization methods. Threads as prepared in Example 1 cross-linked with BDDE were washed in a phosphate buffer or Tris buffer solution at pH 10. Some of the solutions further contained 1 mM ascorbic acid, 10 mM ascorbic acid, 100 mM ascorbic acid, 1 M ascorbic acid, 10 mM vitamin E, and 50 mM Na₃PO₄. The threads were then sterilized using standard e-beam techniques at 4 kGy or 20 kGy. In some embodiments, the temperature of the thread can be altered prior to sterilizing. In some embodiments, the temperature is reduced of the thread to about -20 °C. In some embodiments, the thread is just below 5 °C after sterilizing.

Using the steps disclosed above, threads can be prepared using any one of the processes disclosed below.

### Example 1A: Process 1

Threads can be prepared using the following steps:
1. Cross-linking;
2. Rinsing;
3. Sizing;
4. Drying;
5. Formulating the cross-linked HA with binder;
6. Deaerating;
7. Extruding;
8. Thread Drying;
9. Attaching to a needle; and
10. Sterilizing.

### Example 1B: Process 2

Threads can also be prepared using the following steps:
1. Cross-linking;
2. Rinsing;
3. Sizing;
4. Drying;
5. Formulating the cross-linked HA with binder;
6. Deaerating and autoclaving;
7. Extruding;
8. Thread Drying;
9. Attaching to a needle; and
10. Sterilizing (optional).

### Example 1C: Process 3

Threads can also be prepared using the following steps:
1. Cross-linking;
2. Extruding;
3. Thread Drying;
4. Rinsing;
5. Thread Drying;
6. Attaching to a needle; and
7. Auto-claving.

### Example 1D: Process 4

Threads can also be prepared using the following steps:
1. Cross-linking;
2. Rinsing;
3. Sizing and Formulating the cross-linked HA with binder;
4. Drying;
5. Deaerating;
6. Extruding;
7. Thread Drying;
8. Attaching to a needle; and
9. Sterilizing.

### Example 1E: Process 5

Threads can also be prepared using the following steps:
1. Cross-linking;
2. Rinsing;
3. Sizing;
4. Precipitating from ethanol and drying;
5. Formulating the cross-linked HA with binder;
6. Deaerating;
7. Extruding;
8. Thread Drying;
9. Attaching to a needle; and
10. Sterilizing (optional).

### Example 2: Washing (Re-Hydrating) and Re-Drying the Thread

The dry threads can be washed with an aqueous solvent to remove any contaminants, such as unreacted cross-linking agent. The washing can be performed by various methods, such as submersion in an aqueous solvent or by using a concurrent flow system by placing the thread in a trough at an incline and allowing an aqueous solvent to flow over the thread. In addition, the thread, once it is rehydrated, can be stretched prior to re-drying. The stretching can be performed by the means described above in Example 1. The rehydrated and washed thread is then re-dried to provide the dry thread. The re-drying is typically performed under ambient conditions (i.e. ambient temperature and/or pressure) for from about 8 hours to about 24 hours or until the dry thread has a percent hydration of less than about 30%. The thread can be washed several times (e.g. 10 or more times) without losing its structural integrity. Over the course of multiple washing cycles the overall length of the thread can be increased by between about 25% to about 100%.

### Example 3: Sample Threads

Sample threads prepared from the methods of Example 1 are provided in Table 1 below.

**Table 1**

| **Sample Thread** | **Gel** | **Pre-size** | **% HA Solids** | **% NXL HA** | **Deaeration** | **Extrusion Nozzle** |
|---|---|---|---|---|---|---|
| 113 | 8/40 | 20G-taper | 15 | 20 | 4X | 20G |
| 114 | 8/40 | 20G-taper | 15 | 20 | 2X | 20G |
| 115 | 8/40 | 20G-taper | 15 | 20 | 6X | 20G |
| 116 | 8/40 | 20G-taper | 15 | 20 | 6X, Overnight | 20G |
| 117 | 10/40 | 16G/20G-taper | 15 | 20 | 6X | 20G, 19G |
| 118 | 10/40 | 16G/20G-taper | 15 | 40 | 6X | 20G, 19G |
| 119 | 10/40 | 16G/20G-taper | 15 | 40 | 10X | 20G, 19G |
| 120 | 10/40 | 16G/20G-taper | 15 | 20 | 10X | 20G, 19G |
| 121 | 10/40 | 16G/20G-taper | 10 | 50 | 3X | 19G, 18G |
| 122 | 10/40 | 16G/20G-taper | 10 | 50 | 6X | 19G, 18G |
| 123 | 10/40 | 16/18/20-taper | 10 | 50 | 6X | 19G, 18G |
| 124 | 10/40 | 16/18/20-taper | 15 | 20 | 6X | 20G, 19G |
| 125 | 10/40 | 16/18/20-taper | 15 | 40 | 6X | 20G, 19G |
| 126 | 10/40 | 16/18/20-taper (2X) | 15 | 40 | 6X | 20G, 19G |
| 127 | 8/40 | 20G-taper | 15 | 20 | 6X | 20G, 19G |
| 128 | 8/40 | | 15 | 20 | 8X | 19G |
| 129 | 8/40 | | 15 | 20 | 8X | 19G |

| | | | | | | |
|---|---|---|---|---|---|---|
| % HA Solids = cross-linked and noncross-linked HA; NXL = noncross-linked HA | | | | | | |

### Example 4: Physical Characteristics of the Threads

Various threads prepared as described above were evaluated for thread density, dry thread circularity and diameter. Thread density was determined by measuring the weight of a thread of a measured length and calculating the ratio of weight to length. Dry thread circularity (W:T) and diameter (D) were determined by sectioning threads axially and measuring the shortest (W) and longest diameter (T) for a given cross-section. Circularity or aspect ratio is the ratio of W:T. Thread diameter (D) is an average of short (W) and long (T) diameters. The average thread density for threads made with 20G, 19G and 18G needles were 1.78 mg/in (n=7), 2.61 mg/in (n=8), and 2.07 mg/in (n=3), respectively.

### Example 5: Comparison of Ultimate Tensile Strength of Different Threads

Various threads prepared as described above were tested for tensile strength using a force gauge (e.g. Digital Force Gauge by Precision Instruments or Chatillon). Failure was determined by weight at which the thread broke. The ultimate tensile strength was calculated by dividing the tensile force/failure load by the cross-sectional area of the thread. The average failure load (in pounds) for unsterilized threads made with 20G and 19G needles were from about 0.420 lb to about 1.172 lb. The average failure load (in pounds) for e-beam sterilized threads made with 20G, 19G and 18G needles were from about 0.330 lb to about 0.997 lb. The average elongation (in inches) for unsterilized threads made with 20G and 19G needles were from about 0.028 inches to about 0.192 inches. The average elongation (in inches) for e-beam sterilized threads made with 20G, 19G and 18G needles were from about 0.021 inches to about 0.078 inches. The average tensile strength (in psi) for unsterilized threads made with 20G and 19G needles were from about 3236 psi to about 19922 psi. The average tensile strength (in psi) for e-beam sterilized threads made with 20G and 19G needles were from about 1943 psi to about 12859 psi.

### Example 6: Swelling Data

The mass swelling ratio, as represented by V2/V0, is the ratio of the swollen gel weight relative to the fully dried gel weight (Tables 2 and 3).

The diameter swelling ratio (ratio of hydrated thread to dry thread) of threads extruded from a 20G extrusion nozzle having an average dry thread diameter of 0.0132 inches was calculated. Each thread tested had a diameter swelling ratio of 1.5 or more, with the average diameter swelling ratio being 1.55.

**Table 2**

| **Threads Swollen in Water (V2/V0)** | | | | | | | | |
|---|---|---|---|---|---|---|---|---|
| **HA/BDDE** | **5/40** | **6/40** | **7/40** | **8/40** | **9/40** | **10/40** | ***10/10** | ***10/100** |
| **Avg** | 682.7 | 396.7 | 306.2 | 494.7 | 85.3 | 183.3 | 967.5 | 56.1 |
| **Std** | | | | 322.8 | | 188.4 | | 11.9 |
| **Min** | | | | 122.3 | | 50.1 | | 54.5 |
| **Max** | | | | 696.2 | | 696.2 | | 76.4 |

| | | | | | | | | |
|---|---|---|---|---|---|---|---|---|
| *Reference | | | | | | | | |

**Table 3**

| **Threads Swollen in PBS (V2/V0)** | | | | | |
|---|---|---|---|---|---|
| **HA/BDDE** | ***10/10** | ***10/20** | **10/30** | **10/40** | ***10/100** |
| **Avg** | 71.6 | 45.2 | 38.4 | 37.3 | 23.7 |
| **Std** | 5.2 | 0.9 | 1.0 | 4.8 | 1.4 |
| **Min** | 62.8 | 44.6 | 37.7 | 32.0 | 22.4 |
| **Max** | 78.3 | 45.8 | 39.1 | 54.4 | 25.3 |

| | | | | | |
|---|---|---|---|---|---|
| *Reference | | | | | |

### Example 7: Enzymatic Degradation of the Threads with Hyaluronidase

Threads were incubated with hyaluronidase at 1 mg/mL in 1 mL of buffer (100 mM sodium acetate, pH 4.5, 150 mM NaCl) at 37 °C; control threads were are not in the presence of hyaluronidase. At various times, 40 µL aliquots of supernate were withdrawn and assayed by the Carbazole Assay (Cesaretti, M, et al., Carbohydrate Polymers 54: 59-61 (2003) for hyaluronic acid. Vertical arrows correspond to the addition of fresh 1 mg/mL of hyaluronidase. The supernates were replaced with fresh 1 mg/mL enzyme in buffer. For threads A-F: A = 10/40@15/20 CaCl₂; B = 10/40@15/40; C = 10/40@15/20; D = 10/40@10/50; E = 8/40@15/20 H₂O wash; F = 8/40@15/20; G = Control for 10/40@15/20 CaCl₂; H = Control for 10/40@15/40; I = Control for 10/40@15/20; J = Control for 8/40@15/20. Details of these enzymatic degradations represented are in **Fig. 12** and Table 4. This shows that the control threads, which are not in the presence of hyaluronidase, do not degrade over the time monitored, whereas the threads which are in the presence of hyaluronidase do degrade.

**Table 4: Degradation Rate (Slope of linear fit of first five data points)**

| | A | B | C | D | E | F | G | H | I | J |
|---|---|---|---|---|---|---|---|---|---|---|
| Degradation Rate* | 0.014 | 0.011 | 0.010 | 0.007 | 0.041 | 0.023 | 0.00 | 0.00 | 0.00 | 0.00 |

| | | | | | | | | | | |
|---|---|---|---|---|---|---|---|---|---|---|
| *mg HA/day | | | | | | | | | | |

### Example 8: Palpation Data

A blinded evaluator palpates and scores all sample sites per animal and records the value on pre-printed data sheets. The data was compiled and averaged based on the sample site. The evaluators' scores are 0=no tactile signs of implant, 1= slight tactile sign of implant, and 2= high tactile sign of implant. Implant tactile scoring (qualitative palpation) was performed per protocol on test subjects at pre-specified intervals spanning the duration of the study, measuring the surface dermal response post implantation. The response measured was the length continuity of the implant. For example, if palpating from two points, and the implant feels continuous, with no breaks, then that would rate a "2", if the implant feels longitudinally intermittent then that would rate a "1", and if no implant is detected, then the score would rate "0". A plot of number of days vs. average palpation score is shown in **Fig. 13****.**

### Example 9: Histological Studies

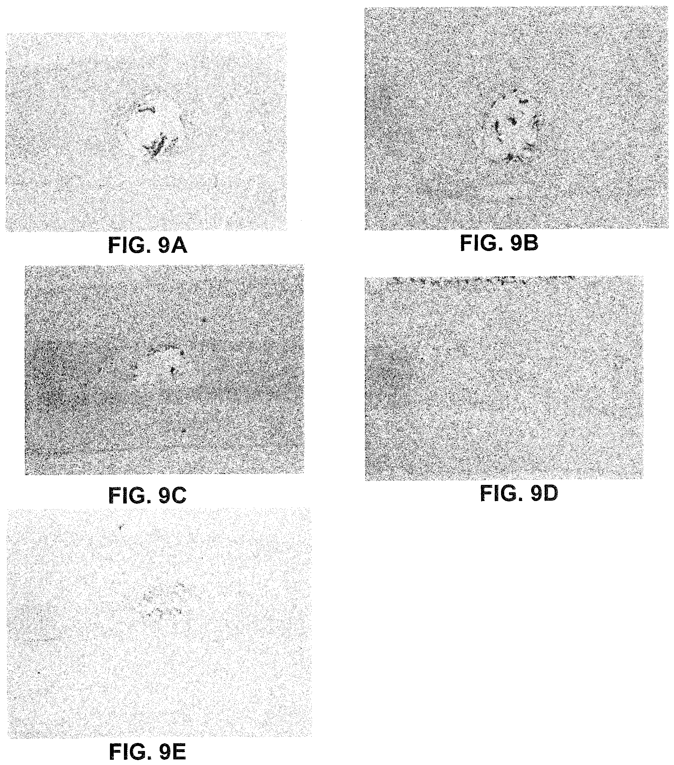

Tissue samples containing thread implants were fixed in an acid-formalin ethanol fixative before embedding in methyl methacrylate. Histological sections were stained with hematoxylin and eosin (H&E) as well as Alcian blue with neutral red to visualize cellular responses and implant presence, respectively. The 1-month, 2-month, 3-month, 6-month and 9-month histology images of 8/40 @ 15/20 formulation are shown in **Fig. 9A, 9B, 9C, 9D, 9E** and **9E****.** The samples were similar in appearance indicating that the threads were persisting *in vivo.*

### Example 10: Studies of Hyaluronic Acid Threads in Rabbits

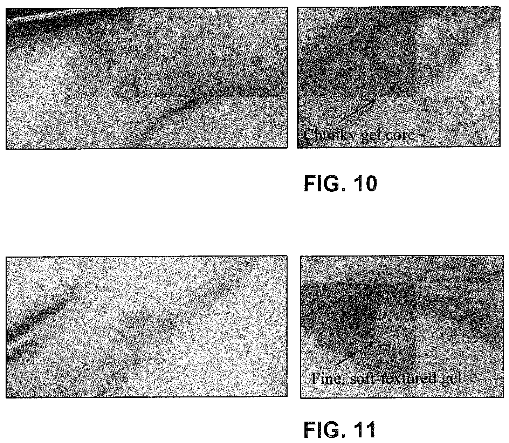

The *in vivo* performance, including persistence and biological response, of thread formulations was evaluated in a chronic rabbit study. Thread formulations described herein were implanted in the dorsal dermis of New Zealand White rabbits and evaluated at 1-week, 1-month, 2-month, 3-month, 6-month, and 9-month time points. During the in-life phase, implant sites were evaluated for implant presence via palpation and a tactile scoring system (See Example 8). At the scheduled time intervals, animals were euthanized and implant sites harvested for gross and histological evaluation. A subset of implant sites were cross-sectioned axially with a scalpel and evaluated under microscope for the visual presence of implant material in the dermal tissue. Pressure was applied to the implant site to assess if implants were well-integrated within the tissue or could be extruded from the implant site. The remaining subset of implant sites were fixed and processed for histological evaluation (see Example 9). The 1-month and 2-month histology images of 8/40 @ 15/20 formulation are shown in **Figs. 10** and **11****,** respectively, showing that the thread is still present.

### Example 11: Treatment of Wrinkles with Hyaluronic Acid Threads

Hypodermic needles (22 to 25 Ga) are affixed with single or double strands of hyaluronic acid threads (cross-linked with BDDE), ranging from thicknesses of 0.004 in to 0.008 in. The samples are e-beam sterilized by NuTek Corp. at 29 kGy. The needle pulls the attached thread or threads into the skin. Wrinkles which are treated are wrinkles in the naso-labial fold, peri-orals, peri-orbitals, frontalis (forehead), and glabellar. The needle would then pull the thread through the skin such that the thread is located where the needle was previously inserted. More than one thread can be used to treat the wrinkles in order to achieve the desired fill effect (two to four or more threads). The wrinkle is visibly lessened upon thread hydration.

### Example 12: Lip Augmentation

A patient may be implanted with HA threads for lip enhancement, either contouring and/or plumping. The patient may receive topical anesthetic on the face, but it is not applied specifically to the lips according to the following procedure:
- Peal open the pouch and remove the sterile tray holding the HA (hyaluronic acid) threads.
- Using sterile gloves or a sterile implement such as forceps, remove the desired HA thread from the tray.
- Insert the sharp end of the needle into one margin of the intended treatment area.
- Translate the needle within the skin under or near the intended treatment area. If the needle is not in a desired location at any point, gently retract the needle and reinsert to correct the location.
- Exit the skin at the opposing margin of the intended treatment area using the sharp end of the needle. If the needle is not in the desired location, gently retract the needle and reinsert to correct the location.
- Upon confirming the desirable location of the needle, swiftly pull the needle distally, pulling the thread into place within the skin.
- Using sterile surgical scissors or scalpel, cut the excess thread protruding from the skin on both margins of the treatment area. This effectively separates the needle, which should be discarded appropriately.

Areas of enhancement include the vermillion border (or white roll) for lip effacement and contouring, the wet-dry mucosal junction for increasing fullness. Other techniques include more diffuse infiltration of the orbicularis oris muscle. The attending clinician is able to select the location of the thread placement, the number of threads and the size of the threads depending on desired effect. It is contemplated that each area is treated with 1 to 2 threads wherein each thread has a diameter of anywhere from 200 microns to about 500 microns when the thread is dry. After hydration, it is contemplated that the thread has a diameter of from 0.5 millimeters to about 5 millimeters.

### Example 13: NMR study to determine the ratio of BDDE derivative to HA in cross-linked hydrogels

An NMR study was undertaken to determine the ratio of 1,4-butanediol diglycidyl ether derivative (BDDE) to the disaccharide subunit of hyaluronic acid (HA) in cross-linked HA hydrogels. Hydrogels were made at HA concentrations of 8 and 10 percent, cross-linked with 3.2 and 4 percent BDDE, respectively. The gels were rinsed extensively to remove residual BDDE, digested with hyaluronidase, and dried. The resulting powders were dissolved in D₂O and analyzed by proton NMR. The ratio of BDDE to the disaccharide subunit of HA was determined by comparing the peak from the inner methylene hydrogens of 1,4-butanediol at 1.6 ppm to the acetyl methyl group of N-acetylglucosylamine at 2.0 ppm. At equal molar amounts of BDDE and disaccharide subunit these peak areas should integrate to 4 and 3, respectively. The results with the 8% HA hydrogel gave peak areas integrating to 0.75 and 3, respectively, which corresponds to about 0.19 mole of BDDE per mole disaccharide subunit. The results with the 10% HA hydrogel gave peak areas integrating to 0.72 and 3, respectively, which corresponds to about 0.18 mole of BDDE per mole disaccharide subunit.

The experiment with the 8/40 formulation was repeated with second batch of gel and gave results of 0.217 mole of BDDE per mole disaccharide subunit. The average percent BDDE in the 8/40 gel is therefore 20% ± 2% S.D. Table 5, below, shows additional formulations.

**Table 5**

| **Gel Formulation** | **% BDDE input (by wt)** | **% substituted BDDE*** | **% cross-linked BDDE*** | **% pendant BDDE*** |
|---|---|---|---|---|
| 12/40 | 40% | 35.2 | 8.5 | 26.7 |
| 12/20 ** | 20% | 19.1 | 4.9 | 14.2 |
| 12/15 ** | 15% | 17.2 | 3.5 | 13.7 |

| | | | | |
|---|---|---|---|---|
| * mol% ** Reference | | | | |

### Example 14: Effect of cross-linking on in vivo persistency of the thread - Animal Data Comparison

Sterilized hyaluronic acid threads produced via the following methods were placed in an animal and evaluated for *in vivo* persistency.

### Part 14A

HA threads were prepared from a 10% w/w hyaluronic acid (MW = 1.47 MDa) gel formulated in 10 mM sodium bicarbonate buffer (pH 10) and cross-linked with 10% (relative to HA mass) 1,4-butanediol diglycidyl ether (BDDE). Threads in Test Articles 1 and 2 were rinsed in Tris buffer (50 mM Tris, 150 mM NaCl) containing 100 mM ascorbic acid, threads in Test Articles 3 and 4 were rinsed in Tris buffer (50 mM Tris, 150 mM NaCl) containing 100 mM Vitamin E, threads inTest Articles 5 and 6 were rinsed in water prior to final drying (air dried). Threads in Test Articles 7 and 8 were prepared in 10 mM Tris buffer (pH 7) rather than 10 mM sodium bicarbonate buffer (pH 10) and were rinsed in water prior to final drying. Dried threads were cylindrical in shape, 0.006-0.008" in diameter and 1.0-1.5" in length. Hypodermic, stainless steel, 27-gauge, thin wall Keith needles were used in all test articles. Needles were 2 ½" in length and had single bevel tips. Cross-linked HA threads were attached to the needle via mechanical crimp. Test Articles were terminally sterilized via e-beam irradiation. Test Articles 1 and 3 received an irradiation dose of 4 kGy. Test Articles 2 and 4 received an irradiation dose of 20 kGy. Test Articles 6 and 8 received an irradiation dose of 20 kGy frozen. Test Articles 5 and 7 were processed in an aseptic-like environment and were not terminally sterilized.

The results of the *in vivo* persistence study (utilizing 10 Sprague Dawley rats) indicated that all of the threads in Test Article 2 were undetectable by one week. The implanted threads of Test Articles 1, 3, 4 and 6 were largely gone by one week with only small numbers of intracellular particles found within small cellular infiltrates at the one-week timepoint. More residual material was seen in the Test Articles 3 and 4 treated sites than Test Article 1 treated sites at one week, which suggests that the resorption rate was slower for those threads. None of the implanted material of Test Articles 1, 2, 3, or 4 was seen at 3 weeks. In addition, all threads from Test Articles 5, 6, 7, and 8 were undetectable by Day 30.

### Part 14B

A 0.75% w/w hyaluronic acid (1.7 MDa) solution was prepared by dissolving the HA in 10 mM TRIS buffer (pH 7). Butanediol diglycidyl ether (BDDE) was added to the HA solution and the solution was stirred overnight. The ratio of BDDE to HA was 2:1. The substantially cross-linked HA solution was then dialyzed against excess deionized water using a dialysis membrane with a molecular weight cut-off of about 12 to about 14 KDa. The dialyzed solution was then lyophilized to obtain dry substantially cross-linked hyaluronic acid. The dry cross-linked hyaluronic acid (2.0 g) was exposed to 25 KGy e-beam (Irradiation temperature 1 - 3 °C), formulated to 16% solids (w/w) in 10 mM TRIS buffer (pH 7.00), extruded and dried for about 48 hrs at ambient temperature.

Test Article 9 was irradiated after cross-linking at 25kGy, 0.020" diameter, processed in an aseptic-like environment and non-terminally sterilized. Test Article 10 was irradiated after cross-linking at 25kGy, 0.010" diameter, processed in an aseptic-like environment and non-terminally sterilized. Test Article 11 was 0.020" in diameter, processed in an aseptic-like environment and non-terminally sterilized. Test Article 12 was irradiated after cross-linking at 25kGy, 0.020" diameter, processed in an aseptic-like environment and non-terminally sterilized. Test Article 13 was irradiated after cross-linking at 25kGy, 0.010" diameter, processed in an aseptic-like environment and non-terminally sterilized. Test Article 14 was 0.020" in diameter, processed in an aseptic-like environment and non-terminally sterilized.

The threads of Test Articles 9-14 were implanted in rabbits. Skin segments containing a total of 20 treated sites were removed from each animal. Select specimens (4/group/timepoint taken from each thread(s) of Test Article 9-14 treated sites) were processed and analyzed. Complete resorption of the threads of Test Articles 9, 10, and 13 occurred by 7 days. Complete resorption of the threads of Test Article 12 occurred between 7 and 30 days. Nearly complete or complete resorption of the threads of Test Article 14 had occurred and partial resorption of the threads of Test Article 11 had occurred by 30 days.

### Part 14C

Six cross-linked threads were prepared according to Example 1. The threads of Test Articles 15-20 were prepared as follows. Cross linked threads of diameter between 0.008 inches and 0.010 inches was made by forming a gel with a concentration of 10% hyaluronic acid and 4% BDDE by weight relative to total composition with the remainder comprised of 0.1 N sodium hydroxide. The ratio of BDDE to HA in the cross-linked gel was 0.18 mole of BDDE per mole disaccharide subunit. After rinsing, presizing, lyophilizing, and combining with noncross-linked binder, the gel was then extruded into a thread form by using a 20G nozzle. The wet thread is then dried for about 48 hrs to provide a dry thread.

**Table 6. Test Article Composition/Processing**

| **Test Articl e** | **Startin g [HA]** | **[BDDE ]** | **Rinse mediu m** | **Presize d** | **Ha solid s in gel** | **% Binde r** | **Formulatio n medium** | **Sterilizatio n** |
|---|---|---|---|---|---|---|---|---|
| **15** | 10% | 40% | PB | 20G | 10% | 50% | H₂O | E-beam |
| **16** | 10% | 40% | PB | 20G | 15% | 20% | H₂O | E-beam |
| **17** | 10% | 40% | PB | 20G | 15% | 20% | 5 mM CaCl H₂O | E-beam |
| **18** | 10% | 40% | PB | 20G | 15% | 40% | H₂O | E-beam |
| **19** | 8% | 40% | PB | 20G | 15% | 20% | H₂O | E-beam |
| **20** | 8% | 40% | H₂O | 20G | 15% | 20% | H₂O | E-beam |

| | | | | | | | | |
|---|---|---|---|---|---|---|---|---|
| PB = 10 mM sodium phosphate buffer, pH 6 | | | | | | | | |

Test Articles were successfully assessed for degradation by macroscopic observation and by histology analysis. All Test Articles were identifiable by macroscopic observation through 90 days, but with the exception of Test Article 20, identification was inconsistent at 135 and 180 days. Histologically, all Test Articles were identifiable qualitiatively at early time points. Qualitiatively, Test Articles 15, 16, 17, and 18 demonstrated more evidence of degradation than Test Articles 19 and 20. Degradation of Test Article 20 was quantitatively analyzed and by cross-sectional area persisted without significant change at 270 days.

Thus, the more cross-linked thread of this disclosure is more persistent *in vivo* than the less cross-linked thread as can be seen from the above data.

## Claims

1. A composition comprising (i) at least 5 weight % hyaluronic acid, wherein the hyaluronic acid is substantially cross-linked with at least about 15 mole % of a butanediol diglycidyl ether (BDDE) derivative relative to the repeating disaccharide unit of the hyaluronic acid, and (ii) a binder which is noncross-linked hyaluronic acid, wherein the substantially cross-linked hyaluronic acid is cross-linked with at least 30 weight % to 50 weight % of BDDE relative to the weight of the hyaluronic acid.

2. The composition of claim 1, wherein the substantially cross-linked hyaluronic acid is cross-linked with from about 15 to about 25 mole % of the BDDE derivative.

3. The composition of any one of claims 1-2, wherein the substantially cross-linked hyaluronic acid is cross-linked with from about 17 to about 20 mole % of the BDDE derivative.

4. The composition of any one of claims 1-3, wherein the substantially cross-linked hyaluronic acid is cross-linked with about 40 weight % of the BDDE derivative relative to the weight of the hyaluronic acid.

5. The composition of any one of claims 1-4, wherein the composition comprises about 8% hyaluronic acid.

6. The composition of claim 1, wherein the composition comprises about 10% hyaluronic acid.

7. The composition of claim 1, wherein the composition comprises about 12% hyaluronic acid.

8. The composition of any one of claims 1-7, wherein the molecular weight of the hyaluronic acid is from about 0.5 to about 3.0 MDa.

9. The composition of any one of claims 1-8, wherein the cross-linked hyaluronic acid is present in an amount of from about 5 weight % to about 20 weight % based on the total weight of the composition.

10. The composition of any one of claims 1-9, wherein the noncross-linked hyaluronic acid is present in an amount of from about 1 weight % to about 15 weight % based on the total weight of the composition.

11. The composition of claim 10, wherein the noncross-linked hyaluronic acid is present in an amount of from about 2 weight % to about 8 weight % based on the total weight of the composition.

12. The composition of any one of claims 1-9, wherein the composition comprises from about 5 weight % to about 15 weight % cross-linked hyaluronic acid and from about 2 weight % to about 8 weight % noncross-linked hyaluronic acid.

13. A thread comprising the composition of any one of claims 1-12.

14. A kit of parts for use in treating a wrinkle in a patient, comprising the thread of claim 13 and a means for delivery of the thread to a patient which is preferably a needle.

15. The thread of claim 13 or the kit of parts of claim 14 for use in a method of treating a wrinkle in a patient in need thereof, said method comprising:
a) inserting the thread of claim 13 into skin or subcutaneous space of the patient adjacent to or under the wrinkle; and
b) applying the thread adjacent to or under the wrinkle thereby treating the wrinkle.

## Patentansprüche

1. Zusammensetzung, umfassend (i) mindestens 5 Gew.-% Hyaluronsäure, wobei die Hyaluronsäure im Wesentlichen mit mindestens etwa 15 mol-% eines Butandioldiglycidylether (BDDE)-Derivats, relativ zu der Disaccharid-Wiederholungseinheit der Hyaluronsäure, vernetzt ist, und (ii) ein Bindemittel, das nichtvernetzte Hyaluronsäure ist, wobei die im Wesentlichen vernetzte Hyaluronsäure mit mindestens 30 Gew.-% bis 50 Gew.-% BDDE, relativ zum Gewicht der Hyaluronsäure, vernetzt ist.

2. Zusammensetzung gemäß Anspruch 1, wobei die im Wesentlichen vernetzte Hyaluronsäure mit etwa 15 bis etwa 25 mol-% des BDDE-Derivats vernetzt ist.

3. Zusammensetzung gemäß irgendeinem der Ansprüche 1-2, wobei die im Wesentlichen vernetzte Hyaluronsäure mit etwa 17 bis etwa 20 mol-% des BDDE-Derivats vernetzt ist.

4. Zusammensetzung gemäß irgendeinem der Ansprüche 1-3, wobei die im Wesentlichen vernetzte Hyaluronsäure mit etwa 40 Gew.-% des BDDE-Derivats, relativ zum Gewicht der Hyaluronsäure, vernetzt ist.

5. Zusammensetzung gemäß irgendeinem der Ansprüche 1-4, wobei die Zusammensetzung etwa 8 % Hyaluronsäure umfasst.

6. Zusammensetzung gemäß Anspruch 1, wobei die Zusammensetzung etwa 10 % Hyaluronsäure umfasst.

7. Zusammensetzung gemäß Anspruch 1, wobei die Zusammensetzung etwa 12 % Hyaluronsäure umfasst.

8. Zusammensetzung irgendeinem der Ansprüche 1-7, wobei das Molekulargewicht der Hyaluronsäure etwa 0,5 bis etwa 3,0 MDa beträgt.

9. Zusammensetzung irgendeinem der Ansprüche 1-8, wobei die vernetzte Hyaluronsäure in einer Menge von etwa 5 Gew.-% bis etwa 20 Gew.-%, bezogen auf das Gesamtgewicht der Zusammensetzung, vorhanden ist.

10. Zusammensetzung irgendeinem der Ansprüche 1-9, wobei die nicht-vernetzte Hyaluronsäure in einer Menge von etwa 1 Gew.-% bis etwa 15 Gew.-%, bezogen auf das Gesamtgewicht der Zusammensetzung, vorhanden ist.

11. Zusammensetzung Anspruch 10, wobei die nicht-vernetzte Hyaluronsäure in einer Menge von etwa 2 Gew.-% bis etwa 8 Gew.-%, bezogen auf das Gesamtgewicht der Zusammensetzung, vorhanden ist.

12. Zusammensetzung irgendeinem der Ansprüche 1-9, wobei die Zusammensetzung etwa 5 Gew.-% bis etwa 15 Gew.-% vernetzte Hyaluronsäure und etwa 2 Gew.-% bis etwa 8 Gew.-% nicht-vernetzte Hyaluronsäure umfasst.

13. Faden, umfassend die Zusammensetzung gemäß irgendeinem der Ansprüche 1-12.

14. Kit of Parts zur Verwendung in der Behandlung einer Falte bei einem Patienten, umfassend den Faden gemäß Anspruch 13 und ein Mittel zur Abgabe des Fadens an einen Patienten, das vorzugsweise eine Nadel ist.

15. Faden gemäß Anspruch 13 oder Kit of Parts gemäß Anspruch 14 zur Verwendung in einem Verfahren zur Behandlung einer Falte bei einem Patienten mit Bedarf dafür, wobei das Verfahren umfasst:
a) Einsetzen des Fadens gemäß Anspruch 13 in die Haut oder einen subkutanen Raum des Patienten angrenzend an die oder unter der Falte und
b) Applizieren des Fadens angrenzend an die oder unter der Falte, wodurch die Falte behandelt wird.

## Revendications

1. Composition comprenant (i) au moins 5 % en poids d'acide hyaluronique, dans laquelle l'acide hyaluronique est sensiblement réticulé avec au moins environ 15 % en mole d'un dérivé de butanediol diglycidyléther (BDDE) par rapport au motif disaccharidique répétitif de l'acide hyaluronique, et (ii) un liant qui est de l'acide hyaluronique non réticulé, dans laquelle l'acide hyaluronique sensiblement réticulé est réticulé avec au moins 30 % en poids à 50 % en poids de BDDE par rapport au poids de l'acide hyaluronique.

2. Composition selon la revendication 1, dans laquelle l'acide hyaluronique sensiblement réticulé est réticulé avec environ 15 à environ 25 % en mole du dérivé de BDDE.

3. Composition selon l'une quelconque des revendications 1 à 2, dans laquelle l'acide hyaluronique sensiblement réticulé est réticulé avec environ 17 à environ 20 % en mole du dérivé de BDDE.

4. Composition selon l'une quelconque des revendications 1 à 3, dans laquelle l'acide hyaluronique sensiblement réticulé est réticulé avec environ 40 % en poids du dérivé de BDDE par rapport au poids de l'acide hyaluronique.

5. Composition selon l'une quelconque des revendications 1 à 4, dans laquelle la composition comprend environ 8 % d'acide hyaluronique.

6. Composition selon la revendication 1, dans laquelle la composition comprend environ 10 % d'acide hyaluronique.

7. Composition selon la revendication 1, dans laquelle la composition comprend environ 12 % d'acide hyaluronique.

8. Composition selon l'une quelconque des revendications 1 à 7, dans laquelle le poids moléculaire de l'acide hyaluronique est d'environ 0,5 à environ 3,0 MDa.

9. Composition selon l'une quelconque des revendications 1 à 8, dans laquelle l'acide hyaluronique réticulé est présent en une quantité d'environ 5 % en poids à environ 20 % en poids sur la base du poids total de la composition.

10. Composition selon l'une quelconque des revendications 1 à 9, dans laquelle l'acide hyaluronique non réticulé est présent en une quantité d'environ 1 % en poids à environ 15 % en poids sur la base du poids total de la composition.

11. Composition selon la revendication 10, dans laquelle l'acide hyaluronique non réticulé est présent en une quantité d'environ 2 % en poids à environ 8 % en poids sur la base du poids total de la composition.

12. Composition selon l'une quelconque des revendications 1 à 9, dans laquelle la composition comprend environ 5 % en poids à environ 15 % en poids d'acide hyaluronique réticulé et environ 2 % en poids à environ 8 % en poids d'acide hyaluronique non réticulé.

13. Fil comprenant la composition selon l'une quelconque des revendications 1 à 12.

14. Kit de pièces à utiliser dans le traitement d'une ride chez un patient, comprenant le fil de la revendication 13 et un moyen de fourniture du fil à un patient qui est de préférence une aiguille.

15. Fil selon la revendication 13 ou kit de pièces selon la revendication 14 à utiliser dans un procédé de traitement d'une ride chez un patient qui en a besoin, ledit procédé comprenant les étapes :
a) d'insertion du fil de la revendication 13 dans la peau ou l'espace sous-cutané du patient adjacent à la ride ou disposé en dessous de celle-ci ; et
b) d'application du fil adjacent à la ride ou en dessous de celle-ci en sorte de traiter la ride.
